# EUROPÄISCHE PATENTANMELDUNG

(11) **EP 3 984 563 A1**
(43) Veröffentlichungstag der Anmeldung: **20.04.2022**
(21) Anmeldenummer: 21202938.3
(22) Anmeldetag: 15.10.2021
(51) Int. Cl.: A61L 2/26, A61B 50/30

(54) **VERSCHLUSSVORRICHTUNG FÜR STERILBEHÄLTER SOWIE STERILBEHÄLTER MIT EINER DERARTIGEN VERSCHLUSSVORRICHTUNG**

(30) Priorität: 16.10.2020 DE 102020127370
(71) Anmelder: Innovations Medical GmbH, 78532 Tuttlingen (DE)
(72) Erfinder: Kreidler, Jochen, 78532 Tuttlingen (DE)
(74) Vertreter: Daub, Thomas

(57) **Zusammenfassung**

Die Erfindung geht aus von einer Verschlussvorrichtung für Sterilbehälter, mit zumindest einer schwenkbar gelagerten Verschlussklappe (14a; 14b), mit zumindest einem zumindest in einer Schließstellung mit der Verschlussklappe (14a; 14b), insbesondere form- und/oder kraftschlüssig, zusammenwirkenden Rastelement (16a; 16b), insbesondere einem schwenkbar gelagerten Spannhebel, und mit zumindest einer zumindest eine Befestigungsschnittstelle (18a, 44a; 18b, 44b) zu einem Befestigen an dem Sterilbehälter aufweisenden Halteeinheit (20a; 20b) zu einem Halten der, insbesondere schwenkbar an der Halteeinheit (20a; 20b) gelagerten, Verschlussklappe (14a; 14b) und/oder des Rastelements (16a; 16b) an dem Sterilbehälter.

Es wird vorgeschlagen, dass die Halteeinheit (20a; 20b) zumindest ein, insbesondere translatorisch, beweglich gelagertes Halteelement (22a, 24a; 22b, 24b) aufweist, das separat zur Verschlussklappe (14a; 14b) ausgebildet ist und derart beweglich gelagert ist, dass das Halteelement (22a, 24a; 22b, 24b) zumindest während eines Überführens der Verschlussklappe (14a; 14b) in oder aus der Schließstellung bewegbar ist.

## Beschreibung

### Stand der Technik

Die Erfindung betrifft eine Verschlussvorrichtung für Sterilbehälter nach dem Oberbegriff des Anspruchs 1 sowie einen Sterilbehälter mit einer derartigen Verschlussvorrichtung.

In EP 2 559 395 B1 ist bereits eine Verschlussvorrichtung für Sterilbehälter, mit zumindest einer schwenkbar gelagerten Verschlussklappe, mit zumindest einem zumindest in einer Schließstellung mit der Verschlussklappe, insbesondere form- und/oder kraftschlüssig, zusammenwirkenden Rastelement, insbesondere einem schwenkbar gelagerten Spannhebel, und mit zumindest einer zumindest eine Befestigungsschnittstelle zu einem Befestigen an dem Sterilbehälter aufweisenden Halteeinheit zu einem Halten der, insbesondere schwenkbar an der Halteeinheit gelagerten, Verschlussklappe und/oder des Rastelements an dem Sterilbehälter, vorgeschlagen worden.

Die Aufgabe der Erfindung besteht insbesondere darin, eine gattungsgemäße Verschlussvorrichtung und/oder einen gattungsgemäßen Sterilbehälter mit verbesserten Eigenschaften hinsichtlich eines Betätigungskomforts und/oder einer Verschlussspannkraft bereitzustellen. Die Aufgabe wird erfindungsgemäß durch die Merkmale des Anspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Verschlussvorrichtung für Sterilbehälter, mit zumindest einer schwenkbar gelagerten Verschlussklappe, mit zumindest einem zumindest in einer Schließstellung mit der Verschlussklappe, insbesondere form- und/oder kraftschlüssig, zusammenwirkenden Rastelement, insbesondere einem schwenkbar gelagerten Spannhebel, und mit zumindest einer zumindest eine Befestigungsschnittstelle zu einem Befestigen an dem Sterilbehälter aufweisenden Halteeinheit zu einem Halten der, insbesondere schwenkbar an der Halteeinheit gelagerten, Verschlussklappe und/oder des Rastelements an dem Sterilbehälter.

Es wird vorgeschlagen, dass die Halteeinheit zumindest ein, insbesondere translatorisch, beweglich gelagertes Halteelement aufweist, das separat zur Verschlussklappe ausgebildet ist und derart beweglich gelagert ist, dass das Halteelement zumindest während eines Überführens der Verschlussklappe in oder aus der Schließstellung bewegbar ist. Vorzugsweise ist das Halteelement verschieden von einem beweglich an der Verschlussklappe gelagerten Rastvorsprung ausgebildet. Bevorzugt wird das Halteelement zumindest während eines Überführens der Verschlussklappe in oder aus der Schließstellung relativ zum Sterilbehälter, insbesondere relativ zu einem Behälterdeckel des Sterilbehälters, bewegt, insbesondere translatorisch bewegt. Bevorzugt ist das Halteelement beweglich entlang und/oder um eine Bewegungsachse des Halteelements gelagert, insbesondere relativ zum Sterilbehälter. Bevorzugt wird das Halteelement zumindest während eines Inkontaktkommens oder eines Außerkontaktkommens der Verschlussklappe mit dem Rastelement, insbesondere des Spannhebels, translatorisch bewegt, insbesondere zusammen mit der Verschlussklappe. Insbesondere ist das Halteelement derart am Sterilbehälter gelagert, dass das Halteelement auch unabhängig von einer Bewegung der Verschlussklappe, insbesondere translatorisch, beweglich relativ zum Sterilbehälter ist. Insbesondere ist die Verschlussklappe über die translatorische Lagerung des Halteelements am Sterilbehälter ebenfalls, insbesondere zusätzlich zur schwenkbaren Lagerung der Verschlussklappe, translatorisch relativ zum Sterilbehälter gelagert. Vorzugsweise ist das Halteelement an einer Außenseite des Sterilbehälters, insbesondere eines Behälterdeckels des Sterilbehälters, beweglich gelagert. Bevorzugt ist das Halteelement in einer zumindest im Wesentlichen parallel zu der eine Seitenfläche bildenden Außenseite des Sterilbehälters, insbesondere des Behälterdeckels des Sterilbehälters, verlaufenden Ebene translatorisch beweglich gelagert. Unter "im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist. Vorzugsweise verläuft die Bewegungsachse des Halteelements zumindest im Wesentlichen parallel zu der die Seitenfläche bildenden Außenseite des Sterilbehälters, insbesondere des Behälterdeckels des Sterilbehälters. Insbesondere verläuft die Bewegungsachse des Halteelements zumindest im Wesentlichen senkrecht zu einer Bodenstandfläche, insbesondere eines Behälterunterteils, des Sterilbehälters, insbesondere in einem an dem Behälterunterteil angeordneten Zustand des Behälterdeckels des Sterilbehälters. Der Ausdruck "im Wesentlichen senkrecht" soll hier insbesondere eine Ausrichtung einer Richtung relativ zu einer Bezugsrichtung definieren, wobei die Richtung und die Bezugsrichtung, insbesondere in einer Projektionsebene betrachtet, einen Winkel von 90° einschließen und der Winkel eine maximale Abweichung von insbesondere kleiner als 8°, vorteilhaft kleiner als 5° und besonders vorteilhaft kleiner als 2° aufweist.

Vorzugsweise ist die Verschlussklappe schwenkbar an dem Halteelement gelagert. Insbesondere ist die Verschlussklappe über ein Lagerelement, insbesondere einen Lagerbolzen, einen Lagerfortsatz o. dgl., der Verschlussvorrichtung beweglich um eine Schwenkachse der Verschlussklappe an dem Halteelement gelagert, insbesondere mit dem Halteelement verbunden. Das Halteelement ist vorzugsweise direkt oder indirekt über ein weiteres Halteelement der Halteeinheit mit einem der Verschlussklappe abgewandten Ende oder mit einer der Verschlussklappe abgewandten Seite mit dem Sterilbehälter, insbesondere mit dem Behälterdeckel des Sterilbehälters, verbunden. Das weitere Halteelement kann einteilig mit dem Sterilbehälter, insbesondere mit dem Behälterdeckel, ausgebildet sein oder das weitere Halteelement kann mittels einer form- und/oder kraftschlüssigen Verbindung an dem Sterilbehälter, insbesondere an dem Behälterdeckel, fixiert sein. Unter "einteilig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling. Die Verschlussklappe ist bevorzugt mittels des beweglich gelagerten Halteelements zusätzlich zu einer schwenkbaren Lagerung translatorisch relativ zum Sterilbehälter gelagert. Es ist jedoch auch denkbar, dass das Rastelement schwenkbar an dem Halteelement gelagert ist oder dass die Halteeinheit ein zusätzliches Halteelement, das beweglich gelagert ist, aufweist, an dem das Rastelement beweglich gelagert ist. Es sind insbesondere Ausgestaltungen denkbar, bei denen entweder die Verschlussklappe oder das Rastelement einzeln mittels des Halteelements translatorisch relativ zum Sterilbehälter beweglich gelagert ist, oder die Verschlussklappe mittels des Halteelements und das Rastelement mittels eines zusätzlichen Halteelements der Halteeinheit translatorisch relativ zum Sterilbehälter beweglich gelagert sind. Weitere, einem Fachmann als sinnvoll erscheinende Ausgestaltungen einer individuellen beweglichen Lagerung der Verschlussklappe und/oder des Rastelements, insbesondere zusätzlich zu einer bereits vorhandenen beweglichen Lagerung der Verschlussklappe und/oder des Rastelements, mittels der Halteeinheit sind ebenfalls denkbar. Die Verschlussklappe ist vorzugsweise mittels der Halteeinheit, insbesondere mittels des Halteelements, an dem Behälterdeckel des Sterilbehälters angeordnet. Die Verschlussklappe ist bevorzugt zusammen mit dem Behälterdeckel von dem Behälterunterteil des Sterilbehälters, das mittels des Behälterdeckels auf eine, einem Fachmann bereits bekannte Art und Weise verschließbar ist, abnehmbar. Das Rastelement ist vorzugsweise mittels der Halteeinheit an dem Behälterunterteil des Sterilbehälters angeordnet, insbesondere beweglich am Behälterunterteil gelagert. Es ist jedoch auch denkbar, dass die Verschlussklappe am Behälterunterteil angeordnet ist und das Rastelement an dem Behälterdeckel angeordnet ist. Mittels der Halteeinheit sind vorzugsweise die Verschlussklappe und das Rastelement verliersicher am Sterilbehälter gehalten/angeordnet. Unter "verliersicher gehalten/angeordnet" soll insbesondere eine Verbindung von zumindest zwei Elementen verstanden werden, die lediglich unter einer Aufbringung von einer Trennkraft, die größer ist als eine funktionsbedingt wirkende Kraft zwischen den zwei Elementen oder unter einer Zuhilfenahme von Werkzeugen, wie beispielsweise Schraubenschlüssel, Schraubendreher o. dgl., von Trennwerkzeugen, wie beispielsweise Sägen, Bolzenschneider o. dgl., und/oder chemischen Trennmitteln, wie beispielsweise Lösungsmittel usw., voneinander trennbar sind.

Die Verschlussklappe weist vorzugsweise eine Rastausnehmung auf, in die das Rastelement, insbesondere der Spannhebel, zumindest in einer Schließstellung der Verschlussklappe mit einem Rastfortsatz des Rastelements eingreift. Die Rastausnehmung der Verschlussklappe ist bevorzugt als Materialaussparung in der Verschlussklappe ausgebildet. Die Verschlussklappe umgibt das Rastelement in einer Schließstellung der Verschlussklappe, betrachtet in der zumindest im Wesentlichen parallel zu der die Seitenfläche bildenden Außenseite des Sterilbehälters verlaufenden Ebene zumindest im Wesentlichen vollständig, insbesondere komplett. Vorzugsweise wirken die Verschlussklappe und das Rastelement, insbesondere der Spannhebel, gemäß der in der EP 2 559 395 B1 bereits beschriebenen Art und Weise miteinander zusammen, um den Sterilbehälter zu verschließen. Die Verschlussklappe und das Rastelement, insbesondere der Spannhebel, weisen bevorzugt eine gemäß der Offenbarung der EP 2 559 395 B1 beschriebene Ausgestaltung auf.

Bevorzugt umfasst die Halteeinheit zumindest ein weiteres, insbesondere das bereits zuvor genannte weitere, Halteelement, das zumindest teilweise die Befestigungsschnittstelle aufweist oder bildet. In zumindest einer Ausgestaltung der Verschlussvorrichtung ist das weitere Halteelement vorzugsweise mittels der Befestigungsschnittstelle an dem Sterilbehälter, insbesondere dem Behälterdeckel des Sterilbehälters, befestigt. In einer alternativen Ausgestaltung der Verschlussvorrichtung ist das weitere Halteelement einteilig mit dem Sterilbehälter, insbesondere dem Behälterdeckel, ausgebildet und bildet zumindest teilweise die Befestigungsschnittstelle. In der alternativen Ausgestaltung umfasst das weitere Halteelement bevorzugt zumindest eine Befestigungsaufnahme, insbesondere ein Gewinde oder eine Durchgangsöffnung, zu einer Aufnahme zumindest eines Befestigungselements, insbesondere eines Befestigungsbolzens, wie beispielsweise eine Schraube o. dgl. Das Halteelement ist vorzugsweise translatorisch beweglich an dem weiteren Halteelement gelagert. Es ist jedoch auch denkbar, dass die Halteeinheit unabhängig von dem weiteren Halteelement ausgebildet ist und das Halteelement die Befestigungsschnittstelle aufweist, wobei die Befestigungsschnittstelle einteilig mit einer Lagerungsschnittstelle des Halteelements zu einer, insbesondere translatorisch, beweglichen Lagerung des Halteelements relativ zum Sterilbehälter ausgebildet ist. Weitere, einem Fachmann als sinnvoll erscheinende Ausgestaltungen der Halteeinheit zu einer Befestigung und/oder Lagerung zumindest des Halteelements an dem Sterilbehälter sind ebenfalls denkbar.

Mittels der erfindungsgemäßen Ausgestaltung der Verschlussvorrichtung kann vorteilhaft ein hoher Betätigungskomfort und/oder eine hohe Verschlussspannkraft realisiert werden. Es kann vorteilhaft eine zusätzliche Bewegung der Verschlussklappe realisiert werden, um eine komfortable Überführung der Verschlussklappe in die Schließstellung oder aus der Schließstellung zu ermöglichen, insbesondere infolge einer zusätzlich zur Schwenkbewegung realisierbaren Linearbewegung durch die translatorisch bewegliche Lagerung des Halteelements, an dem die Verschlussklappe schwenkbar gelagert ist. Es kann konstruktiv einfach eine Bewegungsüberlagerung einer Rotations- und einer Translationsbewegung realisiert werden, um ein komfortables Eingreifen oder aus einem Eingriff Bringen des Rastelements und der Verschlussklappe zu ermöglichen. Eine Gefahr, dass während des Sterilisationsprozesses ein Behälterdeckel vom Behälterunterteil abgehoben wird und somit die Dichtheit nicht mehr gewährleistet ist, kann durch die erfindungsgemäße Ausgestaltung vorteilhaft gering gehalten werden.

Des Weiteren wird vorgeschlagen, dass das Halteelement entlang einer, insbesondere der bereits zuvor genannten, Bewegungsachse des Halteelements translatorisch beweglich gelagert ist, die quer, insbesondere zumindest im Wesentlichen senkrecht, zu einer, insbesondere der bereits zuvor genannten, Schwenkachse der Verschlussklappe verläuft. Vorzugsweise ist das Halteelement zumindest relativ zum Sterilbehälter und/oder zum weiteren Halteelement entlang der Bewegungsachse des Halteelements translatorisch beweglich gelagert. Es ist jedoch auch denkbar, dass das Halteelement alternativ oder zusätzlich entlang einer weiteren und/oder um eine weitere Bewegungsachse beweglich gelagert ist, insbesondere zumindest relativ zum Sterilbehälter und/oder zum weiteren Halteelement. Die Schwenkachse der Verschlussklappe verläuft vorzugsweise zumindest im Wesentlichen parallel zu der die Seitenfläche bildenden Außenseite des Sterilbehälters, insbesondere des Behälterdeckels des Sterilbehälters, und/oder zur Bodenstandfläche, insbesondere des Behälterunterteils, des Sterilbehälters, insbesondere in einem an dem Behälterunterteil angeordneten Zustand des Behälterdeckels des Sterilbehälters. Die Verschlussklappe ist bevorzugt an einem einer Oberseite des Behälterdeckels des Sterilbehälters abgewandten Ende des Halteelements an dem Halteelement angeordnet, insbesondere daran schwenkbar gelagert. Das Halteelement umfasst vorzugsweise zumindest eine Lagerausnehmung, insbesondere an dem der Oberseite des Behälterdeckels des Sterilbehälters abgewandten Ende des Halteelements, die zu einer Aufnahme des Lagerelements, insbesondere des Lagerbolzens, zu einer schwenkbaren Lagerung der Verschlussklappe an dem Halteelement ausgebildet ist. Bevorzugt ist eine translatorische Bewegung des Halteelements entlang der Bewegungsachse des Halteelements infolge einer Einwirkung einer Zugkraftkomponente von der Verschlussklappe auf das Halteelement hervorrufbar. Mittels der erfindungsgemäßen Ausgestaltung der Verschlussvorrichtung kann konstruktiv einfach eine hohe Verschlussspannkraft realisiert werden. Es kann konstruktiv einfach eine vorteilhafte Krafteinwirkung der Verschlussklappe auf das Rastelement realisiert werden, die quer zu einer Bewegungsachs der Verschlussklappe ausgerichtet ist. Es kann vorteilhaft eine zusätzliche Bewegung der Verschlussklappe realisiert werden, um eine komfortable Überführung der Verschlussklappe in die Schließstellung oder aus der Schließstellung zu ermöglichen, insbesondere infolge einer zusätzlich zur Schwenkbewegung realisierbaren Linearbewegung durch die translatorisch bewegliche Lagerung des Halteelements, an dem die Verschlussklappe schwenkbar gelagert ist. Es kann konstruktiv einfach eine Bewegungsüberlagerung einer Rotations- und einer Translationsbewegung realisiert werden, um ein komfortables Eingreifen oder aus einem Eingriff Bringen des Rastelements und der Verschlussklappe zu ermöglichen.

Ferner wird vorgeschlagen, dass die Halteeinheit zumindest ein Vorspannelement, insbesondere eine Feder, aufweist, das eine entlang einer Bewegungsachse des Halteelements wirkende Vorspannkraft auf das Halteelement ausübt. Das Vorspannelement ist bevorzugt als Druckfeder, insbesondere als Schraubendruckfeder, ausgebildet. Es ist jedoch auch denkbar, dass das Vorspannelement eine andere, einem Fachmann als sinnvoll erscheinende Ausgestaltung aufweist, wie beispielsweise eine Ausgestaltung als Zugfeder, insbesondere als Schraubenzugfeder o. dgl., als Elastomer, als Fluidzylinder, insbesondere als Öldruckfeder, als Gasfeder od. dgl., als Tellerfeder o. dgl. Vorzugsweise stützt sich das Vorspannelement mit einem Ende an dem Halteelement ab und mit einem weiteren Ende stützt sich das Vorspannelement an dem weiteren Halteelement oder an dem Behälterdeckel des Sterilbehälters ab. Das Vorspannelement bewirkt in der Schließstellung der Verschlussklappe ein Ausüben einer Zugkraft der Verschlussklappe auf das Rastelement, insbesondere in Richtung des Behälterdeckels des Sterilbehälters. Mittels der erfindungsgemäßen Ausgestaltung der Verschlussvorrichtung kann konstruktiv einfach in einer Schließstellung der Verschlussklappe eine das Rastelement in Richtung einer Spannstellung des Rastelements beaufschlagenden Verschlussspannkraft erzeugt werden. Es kann konstruktiv einfach eine vorteilhafte Krafteinwirkung der Verschlussklappe auf das Rastelement realisiert werden, die einem ungewollten Herausbewegen der Verschlussklappe aus der Schließstellung entgegenwirken kann. Es kann vorteilhaft eine unterstützende Wirkung des Vorspannelements zu einer komfortablen Überführung der Verschlussklappe in die Schließstellung ermöglicht werden.

Weiterhin wird vorgeschlagen, dass das Halteelement einen Hohlraum zu einer Aufnahme eines, insbesondere des bereits zuvor genannten, Vorspannelements der Halteeinheit zumindest teilweise begrenzt, wobei das Vorspannelement zumindest teilweise von dem Halteelement umgeben wird und sich an dem Halteelement abstützt. Bevorzugt ist das Vorspannelement räumlich zwischen dem Halteelement und dem Sterilbehälter, insbesondere dem Behälterdeckel des Sterilbehälters, angeordnet oder zwischen dem Halteelement und dem weiteren Halteelement der Halteeinheit angeordnet. Bevorzugt ist das Halteelement mittels des Vorspannelements federvorgespannt in einer Position des Halteelements relativ zum Sterilbehälter, insbesondere relativ zum Behälterdeckel des Sterilbehälters, und/oder relativ zum weiteren Halteelement angeordnet. Mittels der erfindungsgemäßen Ausgestaltung der Verschlussvorrichtung kann konstruktiv einfach ein Schutz des Vorspannelements erreicht werden. Es kann konstruktiv einfach eine Kraftübertragung auf das Halteelement realisiert werden, um eine vorteilhafte Verschlussspannkraft zu realisieren. Es kann vorteilhaft eine unterstützende Wirkung des Vorspannelements zu einer komfortablen Überführung der Verschlussklappe in die Schließstellung ermöglicht werden.

Zudem wird vorgeschlagen, dass die Halteeinheit zumindest ein, insbesondere das bereits zuvor genannte, Vorspannelement zu einem Ausüben einer entlang einer, insbesondere der bereits zuvor genannten, Bewegungsachse des Halteelements wirkenden Vorspannkraft auf das Halteelement und zumindest ein die Befestigungsschnittstelle umfassendes weiteres, insbesondere das bereits zuvor genannte weitere, Halteelement aufweist, wobei das Halteelement und das weitere Halteelement zusammen das Vorspannelement zumindest im Wesentlichen vollständig umschließen. Vorzugsweise ist das Vorspannelement entlang der Bewegungsachse des Halteelements zwischen dem Halteelement und dem weiteren Halteelement angeordnet. Bevorzugt ist das Vorspannelement entlang einer zumindest im Wesentlichen senkrecht zur Bewegungsachse des Halteelements verlaufenden Richtung zwischen dem Halteelement und dem weiteren Halteelement angeordnet. Bevorzugt umgeben das Halteelement und das weitere Halteelement das Vorspannelement entlang einer um die Bewegungsachse des Halteelements verlaufenden Richtung zumindest im Wesentlichen vollständig. Vorzugsweise begrenzen das Halteelement und das weitere Halteelement zusammen einen zylindrischen, insbesondere einen kreiszylindrischen, Hohlraum zu einer Aufnahme des Vorspannelements zwischen dem Halteelement und dem weiteren Halteelement. Das Vorspannelement liegt vorzugsweise mit einem Ende am Halteelement an und mit einem weiteren Ende liegt das Vorspannelement insbesondere am weiteren Halteelement an. Das Vorspannelement ist bevorzugt derart zwischen dem Halteelement und dem weiteren Halteelement angeordnet, dass eine Längsachse und/oder eine Hauptwirkachse des Vorspannelements zumindest im Wesentlichen parallel, insbesondere koaxial, zur Bewegungsachse des Halteelements ausgerichtet ist. Vorzugsweise ist das Halteelement mittels des Vorspannelements federvorgespannt relativ zum weiteren Halteelement angeordnet. Das Halteelement wird mittels des Vorspannelements in eine von der Verschlussklappe abgewandte Richtung mit einer Vorspannkraft beaufschlagt. Eine maximale Bewegungsstrecke des Halteelements relativ zum weiteren Halteelement entlang der Bewegungsachse des Halteelements ist insbesondere kleiner als 20 mm, bevorzugt kleiner als 10 mm und besonders bevorzugt kleiner als 5 mm. Es ist denkbar, dass die Halteeinheit zumindest ein Dichtungselement aufweist, das dazu vorgesehen ist, den durch das Halteelement und das weitere Halteelement begrenzten Hohlraum zumindest staubdicht abzudichten, insbesondere um einem Eindringen von Schmutz in den Hohlraum entgegenzuwirken. Das Dichtungselement kann beispielsweise als Dichtlippe o. dgl. ausgebildet sein, die zumindest zwischen dem Halteelement und dem weiteren Halteelement angeordnet ist. Das Dichtungselement kann als separates Bauteil ausgebildet sein oder das Dichtungselement kann einteilig mit dem Halteelement oder dem weiteren Halteelement ausgebildet sein. Andere, einem Fachmann als sinnvoll erscheinende Ausgestaltungen und/oder Anordnungen des Dichtungselements sind ebenfalls denkbar. Es ist zudem denkbar, dass die Halteeinheit unabhängig bzw. frei von einem Dichtungselement ausgebildet ist. Mittels der erfindungsgemäßen Ausgestaltung der Verschlussvorrichtung kann vorteilhaft eine sichere Anordnung des Vorspannelements zwischen dem Halteelement und dem weiteren Halteelement erreicht werden. Es kann vorteilhaft eine Verletzungsgefahr, insbesondere eine Einklemmgefahr, eines Bedieners gering gehalten werden, insbesondere da das Vorspannelement für einen Bediener zumindest im Wesentlichen unzugänglich zwischen dem Halteelement und dem weiteren Halteelement angeordnet ist. Es kann konstruktiv einfach in einer Schließstellung der Verschlussklappe eine das Rastelement in Richtung einer Spannstellung des Rastelements beaufschlagenden Verschlussspannkraft erzeugt werden. Es kann konstruktiv einfach eine vorteilhafte Krafteinwirkung der Verschlussklappe auf das Rastelement realisiert werden, die einem ungewollten Herausbewegen der Verschlussklappe aus der Schließstellung entgegenwirken kann. Es kann vorteilhaft eine unterstützende Wirkung des Vorspannelements zu einer komfortablen Überführung der Verschlussklappe in die Schließstellung ermöglicht werden.

Des Weiteren wird vorgeschlagen, dass die Halteeinheit zumindest ein Sicherungselement aufweist, das das Halteelement weitestgehend gegen eine Bewegung entlang einer quer, insbesondere zumindest im Wesentlichen senkrecht, zu einer, insbesondere der bereits zuvor genannten, Bewegungsachse des Halteelements verlaufenden Richtung sichert. Das Sicherungselement ist vorzugsweise zu einem form- und/oder kraftschlüssigen Zusammenwirken mit einem weiteren Element, insbesondere einer Führungsnut oder einem Schraubenkopf, der Halteeinheit vorgesehen. Insbesondere ist das Sicherungselement als federelastischer Rastclip, als federelastischer Rasthaken, als federelastischer Rastfortsatz, als Langloch, insbesondere als Randbereich, der das Langloch begrenzt, oder als ein anderes, einem Fachmann als sinnvoll erscheinendes Sicherungselement ausgebildet. Bevorzugt umfasst die Halteeinheit eine Vielzahl an Sicherungselementen, die das Halteelement weitestgehend gegen eine Bewegung entlang der quer, insbesondere zumindest im Wesentlichen senkrecht, zur Bewegungsachse des Halteelements verlaufenden Richtung sichern. Insbesondere umfasst die Halteeinheit zumindest zwei und bevorzugt zumindest vier Sicherungselemente, die das Halteelement weitestgehend gegen eine Bewegung entlang der quer, insbesondere zumindest im Wesentlichen senkrecht, zur Bewegungsachse des Halteelements verlaufenden Richtung sichern, insbesondere infolge eines Zusammenwirkens mit zumindest einem weiteren Element der Halteeinheit, insbesondere mit zumindest einer Führungsnut oder einem Schraubenkopf der Halteeinheit. Vorzugsweise ist das Sicherungselement bzw. sind die Sicherungselemente versetzt zur Bewegungsachse des Halteelements am Halteelement angeordnet, insbesondere einteilig mit dem Halteelement ausgebildet. Mittels der erfindungsgemäßen Ausgestaltung der Verschlussvorrichtung kann vorteilhaft eine sichere Verbindung des Halteelements und des Sterilbehälters oder des Halteelements und des weiteren Halteelements erreicht werden, die eine zuverlässige Bewegung des Halteelements entlang der Bewegungsachse des Halteelements ermöglicht.

Ferner wird vorgeschlagen, dass, insbesondere in zumindest einer Ausgestaltung der Verschlussvorrichtung, die Halteeinheit zumindest ein, insbesondere das bereits zuvor genannte, Sicherungselement aufweist, das an dem Halteelement angeordnet ist, insbesondere einteilig mit dem Halteelement ausgebildet ist, und in eine, insbesondere an einem, insbesondere dem bereits zuvor genannten, weiteren Halteelement der Halteeinheit, angeordnete Führungsnut der Halteeinheit eingreift. Vorzugsweise rastet das Sicherungselement in die Führungsnut ein. Bevorzugt ist das Sicherungselement dazu vorgesehen, das Halteelement gegen eine Bewegung entlang der quer, insbesondere zumindest im Wesentlichen senkrecht, zur Bewegungsachse des Halteelements verlaufenden Richtung relativ zum weiteren Halteelement zu sichern. Die Längserstreckungsachse der Führungsnut verläuft vorzugsweise zumindest im Wesentlichen parallel zur Bewegungsachse des Halteelements. Die Halteeinheit weist bevorzugt zumindest zwei Führungsnuten auf, die an dem weiteren Halteelement angeordnet sind, insbesondere in das weitere Halteelement eingebracht sind. Vorzugsweise sind die Führungsnuten seitlich versetzt zur Bewegungsachse und/oder zu dem Hohlraum zur Aufnahme des Vorspannelements an dem weiteren Halteelement angeordnet. Es ist jedoch auch denkbar, dass die Halteeinheit eine von zwei abweichende Anzahl an Führungsnuten aufweist, wie beispielsweise drei, vier, fünf usw. Führungsnuten. Vorzugsweise sind pro Führungsnut zumindest zwei Sicherungselemente der Halteeinheit vorgesehen. Insbesondere greifen pro Führungsnut zumindest zwei Sicherungselemente in die Führungsnut ein. Mittels der erfindungsgemäßen Ausgestaltung der Verschlussvorrichtung kann vorteilhaft eine sichere Verbindung des Halteelements und des Sterilbehälters oder des Halteelements und des weiteren Halteelements erreicht werden, die eine zuverlässige Bewegung des Halteelements entlang der Bewegungsachse des Halteelements ermöglicht.

Weiterhin wird vorgeschlagen, dass, insbesondere in zumindest einer Ausgestaltung der Verschlussvorrichtung, die Halteeinheit zumindest ein an dem Halteelement angeordnetes Sicherungselement, insbesondere das bereits zuvor genannte zumindest eine Sicherungselement, und zumindest eine, insbesondere an dem weiteren Halteelement der Halteeinheit angeordnete, Führungsnut, insbesondere die zumindest eine bereits zuvor genannte Führungsnut, aufweist, die infolge eines Zusammenwirkens mit dem Sicherungselement zu einer Führung einer Bewegung des Halteelements entlang einer Bewegungsachse des Halteelements vorgesehen ist. Insbesondere wirkt das Sicherungselement derart form- und/oder kraftschlüssig mit der Führungsnut zusammen, dass eine Bewegung des Sicherungselements entlang einer Längserstreckungsachse der Führungsnut durchführbar ist und eine/r Bewegung des Sicherungselements entlang einer quer, insbesondere zumindest im Wesentlichen senkrecht, zur Längserstreckungsachse infolge eines Formschlusses, insbesondere infolge eines Hintergreifens des Sicherungselements von zumindest einem Rand der Führungsnut, entgegengewirkt oder weitestgehend vermieden wird. Vorzugsweise bilden die Sicherungselemente zusammen mit den Führungsnuten eine Lagerungsschnittstelle der Halteeinheit zu einer beweglichen Lagerung des Halteelements relativ zum Sterilbehälter. Mittels der erfindungsgemäßen Ausgestaltung der Verschlussvorrichtung kann konstruktiv einfach eine Führungsfunktion und eine Sicherungsfunktion kombiniert werden. Es kann vorteilhaft eine sichere Verbindung des Halteelements und des weiteren Halteelements erreicht werden, die eine zuverlässige Bewegung des Halteelements entlang der Bewegungsachse des Halteelements ermöglicht.

Zudem wird vorgeschlagen, dass, insbesondere in zumindest einer Ausgestaltung der Verschlussvorrichtung, die Befestigungsschnittstelle zumindest ein als Langloch ausgebildetes Sicherungselement aufweist, das an dem Halteelement angeordnet ist, insbesondere einteilig mit dem Halteelement ausgebildet ist, und mit zumindest einem Befestigungselement der Befestigungsschnittstelle derart zusammenwirkt, dass eine Führung einer Bewegung des Halteelements entlang einer Bewegungsachse des Halteelements erfolgt. Die Befestigungsschnittstelle wird, insbesondere in zumindest einer Ausgestaltung der Verschlussvorrichtung, vorzugsweise von dem Halteelement, insbesondere von zwei einteilig mit dem Halteelement ausgebildeten Langlöchern, dem weiteren Halteelement und dem Befestigungselement, insbesondere von zumindest zwei Befestigungselementen, gebildet. Das/die Befestigungselement/e ist/sind bevorzugt als Befestigungsbolzen, insbesondere als Schraube/n, ausgebildet. Das als Langloch ausgebildete Sicherungselement weist vorzugsweise eine Haupterstreckungsachse auf, die sich zumindest im Wesentlichen parallel zur Bewegungsachse des Halteelements erstreckt. Unter einer "Haupterstreckungsachse" eines Objekts soll insbesondere eine Achse verstanden werden, welche parallel zu einer längsten Kante eines kleinsten geometrischen Quaders verläuft, welcher das Objekt gerade noch vollständig umschließt. Vorzugsweise wirkt ein Randbereich des Langlochs derart mit einem Schraubenschaft des als Schraube ausgebildeten Befestigungselements zusammen, dass eine Führung einer Bewegung des Halteelements entlang einer Bewegungsachse des Halteelements erfolgt. Mittels der erfindungsgemäßen Ausgestaltung der Verschlussvorrichtung kann vorteilhaft eine sichere Verbindung des Halteelements und des Sterilbehälters oder des Halteelements und des weiteren Halteelements erreicht werden, die eine zuverlässige Bewegung des Halteelements entlang der Bewegungsachse des Halteelements ermöglicht.

Des Weiteren wird vorgeschlagen, dass, insbesondere in zumindest einer Ausgestaltung der Verschlussvorrichtung, die Halteeinheit zumindest ein Gleitelement aufweist, das zu einer Gleitlagerung des Halteelements an einem, insbesondere einteilig mit dem Sterilbehälter ausgebildeten, weiteren Halteelement der Halteeinheit vorgesehen ist. Vorzugsweise weist das Gleitelement eine Oberfläche mit reibungsmindernden Eigenschaften auf. Beispielsweise ist denkbar, dass das Gleitelement selbst aus einem Werkstoff mit geringem Reibwert hergestellt ist, wie beispielsweise Graphit, Polyamid, Polyoxymethylen o. dgl., dass das Gleitelement eine Oberflächenbeschichtung aus einem Werkstoff mit geringem Reibwert aufweist oder dass das Gleitelement Gleitfluideinlagerungen oder Aufnahmen für Gleitfluide aufweist. Bevorzugt umfasst die Halteeinheit eine Vielzahl an Gleitelementen, die zu einer Gleitlagerung des Halteelements an dem, insbesondere einteilig mit dem Sterilbehälter ausgebildeten, weiteren Halteelement der Halteeinheit vorgesehen sind. Bevorzugt sind zumindest zwei der Gleitelemente, insbesondere räumlich, zwischen dem Halteelement und dem weiteren Halteelement angeordnet, insbesondere entlang einer quer zur Bewegungsachse des Halteelements verlaufenden Richtung betrachtet. Vorzugsweise sind zumindest zwei weitere der Gleitelemente, insbesondere räumlich, zwischen dem Halteelement und den zwei Befestigungselementen angeordnet, insbesondere entlang einer quer zur Bewegungsachse des Halteelements verlaufenden Richtung betrachtet. Das/die Gleitelement/e weist/weisen vorzugsweise einen kreisringförmigen Querschnitt auf. Es ist jedoch auch denkbar, dass das/die Gleitelement/e einen anderen, einem Fachmann als sinnvoll erscheinenden Querschnitt aufweist/aufweisen, wie beispielsweise sichelförmig, polygonal o. dgl. Bevorzugt ist/sind das Gleitelement/e abgeschirmt an dem Halteelement und/oder an dem weiteren Halteelement angeordnet, wie beispielsweise mittels einer Anordnung zumindest eines Dichtlippenelements zwischen dem Halteelement und dem weiteren Halteelement zu einem Entgegenwirken eines Eindringens von Schmutz. Mittels der erfindungsgemäßen Ausgestaltung der Verschlussvorrichtung kann vorteilhaft eine zuverlässige Bewegung des Halteelements entlang der Bewegungsachse des Halteelements ermöglicht werden.

Zudem wird vorgeschlagen, dass das Halteelement zumindest einen Anschlagsfortsatz zu einer Begrenzung einer maximalen Bewegungsstrecke des Halteelements aufweist, der zu einem Zusammenwirken mit einem weiteren Halteelement, insbesondere dem bereits zuvor genannten weiteren Halteelement, der Halteeinheit oder mit einem Fortsatz, insbesondere des Behälterdeckels, des Sterilbehälters vorgesehen ist. Vorzugsweise bildet der Anschlagsfortsatz eine Anschlagfläche, die mit einer Gegenanschlagfläche des weiteren Halteelements oder des Sterilbehälters, insbesondere des Behälterdeckels, zu einer Begrenzung der maximalen Bewegungsstrecke des Halteelements entlang der Bewegungsachse des Halteelements zusammenwirkt. Bevorzugt ist die Anschlagfläche des Anschlagsfortsatzes einteilig mit einer Abstützfläche des Halteelements, an der sich das Vorspannelement mit einem Ende an dem Halteelement abstützt, ausgebildet. Es ist jedoch auch denkbar, dass die Anschlagfläche des Anschlagsfortsatzes getrennt von der/zusätzlich zu der Abstützfläche ausgebildet ist. Mittels der erfindungsgemäßen Ausgestaltung der Verschlussvorrichtung kann konstruktiv einfach eine maximale Bewegungsstrecke des Halteelements begrenzt werden. Es kann vorteilhaft einem ungewollten Trennen des Halteelements von dem weiteren Halteelement oder von dem Sterilbehälter infolge einer Bewegung des Halteelements entlang der Bewegungsachse des Halteelements entgegengewirkt werden. Es kann vorteilhaft eine sichere Verbindung des Halteelements und des weiteren Halteelements erreicht werden, die eine zuverlässige Bewegung des Halteelements entlang der Bewegungsachse des Halteelements ermöglicht.

Des Weiteren geht die Erfindung aus von einem Sterilbehälter mit zumindest einer erfindungsgemäßen Verschlussvorrichtung. Der Sterilbehälter ist bevorzugt als medizinischer Sterilbehälter ausgebildet. Der Sterilbehälter umfasst vorzugsweise einen, insbesondere den bereits zuvor genannten, Behälterdeckel und ein, insbesondere das bereits zuvor genannte, Behälterunterteil. Das Behälterunterteil ist vorzugsweise mittels des Behälterdeckels auf eine, einem Fachmann bereits bekannte Art und Weise, insbesondere hermetisch, verschließbar. Bevorzugt ist die Verschlussklappe zumindest mittels des Halteelements oder mittels des Halteelements und des weiteren Halteelements am Behälterdeckel angeordnet, insbesondere schwenkbar und translatorisch beweglich am Behälterdeckel gelagert. Das Rastelement, insbesondere der schwenkbar gelagerte Spannhebel, ist vorzugsweise mittels eines zusätzlichen Halteelements der Halteeinheit am Behälterunterteil angeordnet, insbesondere schwenkbar am Behälterunterteil gelagert. Es ist jedoch auch denkbar, dass die Verschlussklappe am Behälterunterteil angeordnet ist und dass das Rastelement, insbesondere der schwenkbar gelagerte Spannhebel, am Behälterdeckel angeordnet ist. Zudem ist alternativ oder zusätzlich denkbar, dass das Rastelement, insbesondere der schwenkbar gelagerte Spannhebel, mittels der Halteeinheit translatorisch beweglich gelagert ist, insbesondere mittels eines federvorgespannt beweglich gelagerten Halteelements der Halteeinheit. Weitere, einem Fachmann als sinnvoll erscheinende Ausgestaltungen und/oder Anordnungen der Verschlussklappe, des Rastelements und/oder der Halteeinheit sind ebenfalls denkbar. Mittels der erfindungsgemäßen Ausgestaltung des Sterilbehälters kann vorteilhaft ein hoher Bedienkomfort realisiert werden. Es kann vorteilhaft eine zusätzliche Bewegung der Verschlussklappe realisiert werden, um eine komfortable Überführung der Verschlussklappe in die Schließstellung oder aus der Schließstellung zu ermöglichen, insbesondere infolge einer zusätzlich zur Schwenkbewegung realisierbaren Linearbewegung durch die translatorisch bewegliche Lagerung des Halteelements, an dem die Verschlussklappe schwenkbar gelagert ist. Es kann konstruktiv einfach eine Bewegungsüberlagerung einer Rotations- und einer Translationsbewegung realisiert werden, um ein komfortables Eingreifen oder aus einem Eingriff Bringen des Rastelements und der Verschlussklappe zu ermöglichen.

Die erfindungsgemäße Verschlussvorrichtung und/oder der erfindungsgemäße Sterilbehälter sollen/soll hierbei nicht auf die oben beschriebene Anwendung und Ausführungsform beschränkt sein. Insbesondere können/kann die erfindungsgemäße Verschlussvorrichtung und/oder der erfindungsgemäße Sterilbehälter zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

### Zeichnungen

Weitere Vorteile ergeben sich aus der folgenden Zeichnungsbeschreibung. In den Zeichnungen sind zwei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: einen erfindungsgemäßen Sterilbehälter mit einer erfindungsgemäßen Verschlussvorrichtung in einer schematischen Darstellung,
- Fig. 2: eine Teilschnittansicht durch den erfindungsgemäßen Sterilbehälter in einer schematischen Darstellung,
- Fig. 3: eine Detailansicht der erfindungsgemäßen Verschlussvorrichtung in einer schematischen Darstellung,
- Fig. 4: eine weitere Detailansicht der erfindungsgemäßen Verschlussvorrichtung in einem zumindest teilweise demontierten Zustand in einer schematischen Darstellung,
- Fig. 5: eine Detailansicht eines Halteelements der erfindungsgemäßen Verschlussvorrichtung in einer schematischen Darstellung,
- Fig. 6: einen alternativen erfindungsgemäßen Sterilbehälter mit einer alternativen erfindungsgemäßen Verschlussvorrichtung in einer schematischen Darstellung,
- Fig. 7: eine Detailansicht einer an dem alternativen erfindungsgemäßen Sterilbehälter angeordneten Halteeinheit der alternativen erfindungsgemäßen Verschlussvorrichtung mit einem abgenommenen Halteelement in einer schematischen Darstellung,
- Fig. 8a: eine Detailansicht des Halteelements der alternativen erfindungsgemäßen Verschlussvorrichtung in einer schematischen Darstellung und
- Fig. 8b: eine weitere Detailansicht des Halteelements der alternativen erfindungsgemäßen Verschlussvorrichtung in einer schematischen Darstellung.

### Beschreibung der Ausführungsbeispiele

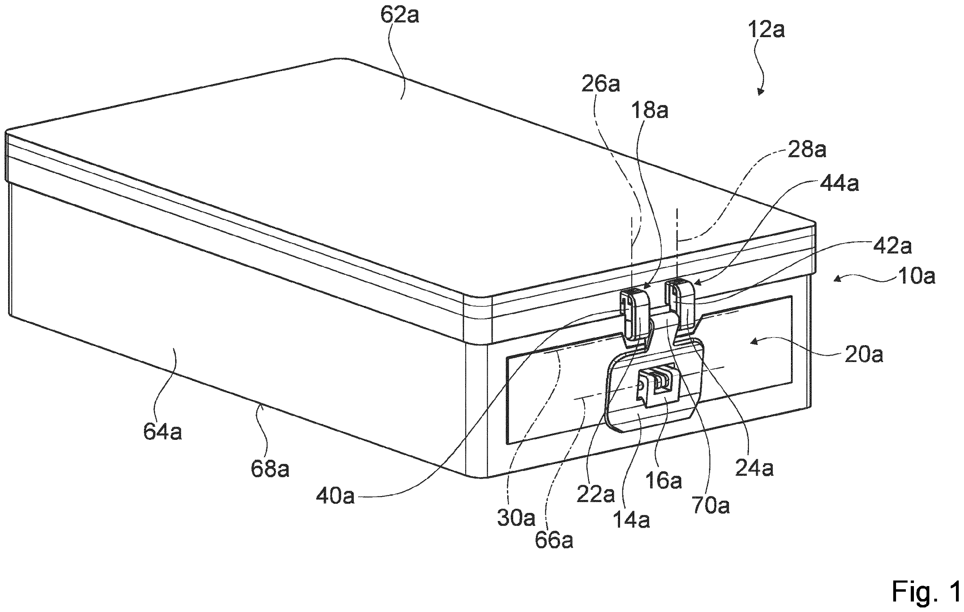

Figur 1 zeigt einen Sterilbehälter 12a mit zumindest einer Verschlussvorrichtung 10a. Der Sterilbehälter 12a ist als medizinischer Sterilbehälter ausgebildet. Es ist jedoch auch denkbar, dass der Sterilbehälter 12a zu einer Verwendung in einem anderen Gebiet vorgesehen ist, in der eine sterile Aufbewahrung von Bauteilen und/oder Werkzeugen sinnvoll oder gewünscht ist. Der Sterilbehälter 12a umfasst einen Behälterdeckel 62a und ein Behälterunterteil 64a. Das Behälterunterteil 64a ist vorzugsweise kastenartig ausgebildet und begrenzt einen Aufnahmeraum. Das Behälterunterteil 64a ist mittels des Behälterdeckels 62a auf eine, einem Fachmann bereits bekannte Art und Weise, insbesondere hermetisch, verschließbar. Das Behälterunterteil 64a ist zur Bildung eines geschlossenen Sterilraums mittels des Behälterdeckels 62a verschließbar. Der Behälterdeckel 62a ist abnehmbar an dem Behälterunterteil 64a angeordnet und mittels umlaufend zwischen dem Behälterdeckel 62a und dem Behälterunterteil 64a angeordneten Dichtungsmitteln luftdicht auf das Behälterunterteil 64a aufsetzbar und mit diesem verbindbar.

Die Verschlussvorrichtung 10a für den Sterilbehälter 12a umfasst zumindest eine schwenkbar gelagerte Verschlussklappe 14a, zumindest ein zumindest in einer Schließstellung mit der Verschlussklappe 14a, insbesondere form- und/oder kraftschlüssig, zusammenwirkendes Rastelement 16a, insbesondere einen schwenkbar gelagerten Spannhebel, und zumindest eine zumindest eine Befestigungsschnittstelle 18a, 44a zu einem Befestigen an dem Sterilbehälter 12a aufweisende Halteeinheit 20a zu einem Halten der, insbesondere schwenkbar an der Halteeinheit 20a gelagerten, Verschlussklappe 14a und/oder des Rastelements 16a an dem Sterilbehälter 12a. Bevorzugt ist das Rastelement 16a als ein, insbesondere schwenkbar gelagerter, Spannhebel gemäß der EP 2 559 395 B1 ausgebildet, so dass in Bezug auf die Ausgestaltung des als Spannhebel ausgebildeten Rastelements 16a zumindest im Wesentlichen auf die Offenbarung der EP 2 559 395 B1 verwiesen wird. Es ist jedoch auch denkbar, dass das Rastelement 16a eine andere, einem Fachmann als sinnvoll erscheinende Ausgestaltung, wie beispielsweise eine Ausgestaltung als translatorisch beweglich und federvorgespannter Rastschieber o. dgl., aufweist. Das als Spannhebel ausgebildete Rastelement 16a ist vorzugsweise aus einer geschlossenen über einem Totpunkt liegenden Spannstellung um eine Schwenkachse 66a des Rastelements 16a schwenkbar in eine geöffnete Schwenkstellung bringbar, wobei die um eine Schwenkachse 30a schwenkbar gelagerte Verschlussklappe 14a in ihrer Schließstellung mit dem als Spannhebel ausgebildeten Rastelement 16a zumindest formschlüssig in Zugverbindung steht, insbesondere analog, wie dies in der EP 2 559 395 B1 offenbart ist.

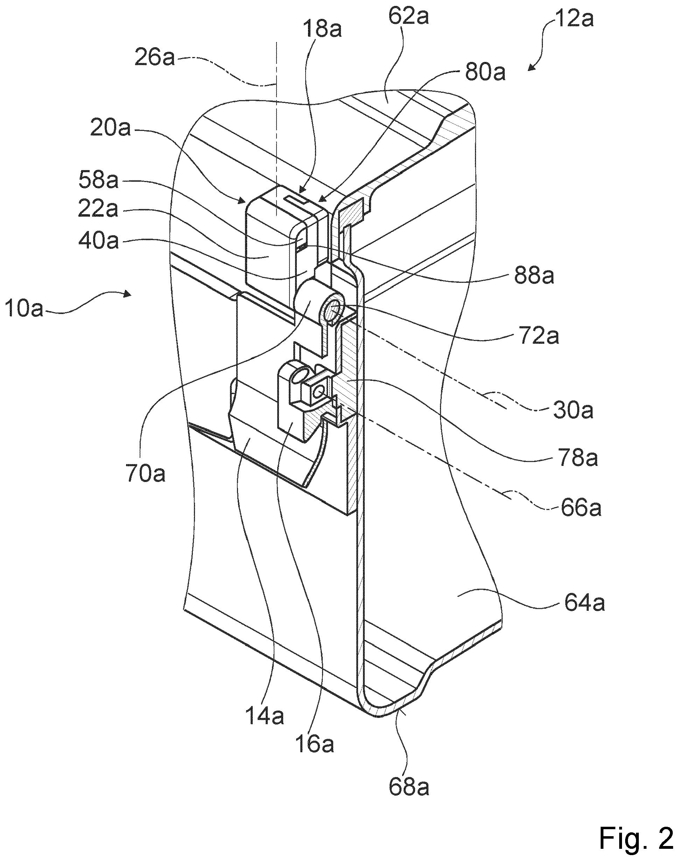

Figur 2 zeigt eine Teilschnittansicht durch den Sterilbehälter 12a im Bereich der Verschlussvorrichtung 10a in einer Schließstellung der Verschlussklappe 14a, in der die Verschlussklappe 14a und das Rastelement 16a form- und/oder kraftschlüssig zusammenwirken, um den Sterilbehälter 12a sicher verschlossen zu halten. Die Verschlussklappe 14a ist vorzugsweise mittels der Halteeinheit 20a an dem Behälterdeckel 62a angeordnet, insbesondere daran schwenkbar gelagert. Das Rastelement 16a ist bevorzugt mittels der Halteeinheit 20a an dem Behälterunterteil 64a, insbesondere an einer Außenseite des Behälterunterteils 64a, angeordnet, insbesondere daran schwenkbar gelagert. Die Schwenkachse 30a der Verschlussklappe 14a verläuft zumindest im Wesentlichen parallel zur Schwenkachse 66a des Rastelements 16a. Die Schwenkachse 30a der Verschlussklappe 14a verläuft vorzugsweise zumindest im Wesentlichen parallel zu einer eine Seitenfläche bildenden Außenseite des Sterilbehälters 12a, insbesondere des Behälterdeckels 62a des Sterilbehälters 12a, und/oder zu einer Bodenstandfläche 68a, insbesondere des Behälterunterteils 64a, des Sterilbehälters 12a, insbesondere in einem an dem Behälterunterteil 64a angeordneten Zustand des Behälterdeckels 62a des Sterilbehälters 12a. Die Verschlussklappe 14a ist bevorzugt an einem einer Oberseite des Behälterdeckels 62a des Sterilbehälters 12a abgewandten Ende der Halteeinheit 20a, insbesondere zumindest zweier Halteelemente 22a, 24a der Halteeinheit 20a, an der Halteeinheit 20a angeordnet, insbesondere daran schwenkbar gelagert.

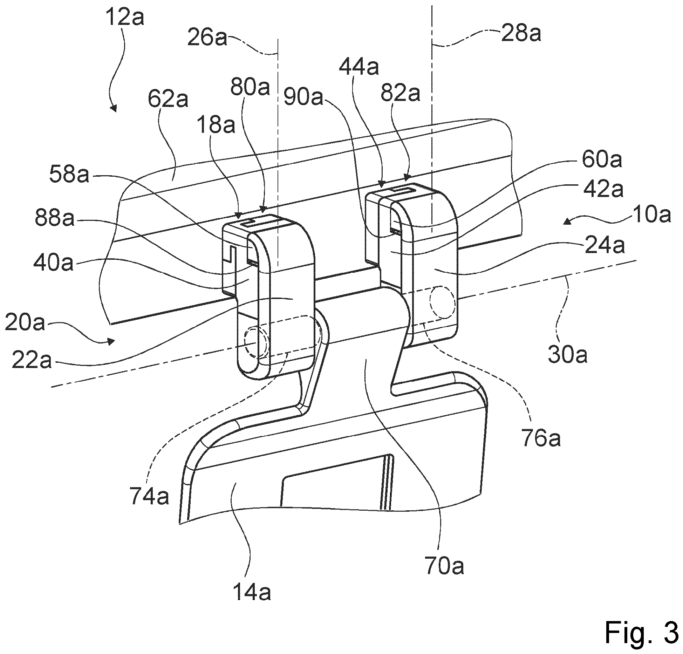

Die Halteeinheit 20a umfasst zumindest ein, insbesondere translatorisch, beweglich gelagertes Halteelement 22a, 24a, das separat zur Verschlussklappe 14a ausgebildet ist und derart beweglich gelagert ist, dass das Halteelement 22a, 24a zumindest während eines Überführens der Verschlussklappe 14a in oder aus der Schließstellung bewegbar ist. Vorzugsweise umfasst die Halteeinheit 20a zumindest, insbesondere genau, zwei Halteelemente 22a, 24a (vgl. Figuren 1 und 3), die translatorisch beweglich gelagert sind und derart beweglich gelagert sind, dass die Halteelemente 22a, 24a zumindest während eines Überführens der Verschlussklappe 14a in oder aus der Schließstellung bewegbar sind. Bewegungsachsen 26a, 28a der Halteelemente 22a, 24a verlaufen zumindest im Wesentlichen parallel zueinander. Ein Lagerfortsatz 70a der Verschlussklappe 14a zu einer Aufnahme eines Lagerelements 72a, insbesondere eines Lagerbolzens, der Verschlussvorrichtung 10a zu einer schwenkbaren Lagerung der Verschlussklappe 14a an der Halteeinheit 20a, insbesondere an den beiden Halteelementen 22a, 24a, ist, betrachtet entlang der Schwenkachse 30a der Verschlussklappe 14a, zwischen den Halteelementen 22a, 24a angeordnet.

Bevorzugt ist die Verschlussklappe 14a zumindest mittels der Halteelemente 22a, 24a oder mittels der Halteelemente 22a, 24a und weiteren Halteelementen 40a, 42a der Halteeinheit 20a am Behälterdeckel 62a angeordnet, insbesondere schwenkbar und translatorisch beweglich am Behälterdeckel 62a gelagert (vgl. Figuren 2 bis 4). Das Rastelement 16a, insbesondere der schwenkbar gelagerte Spannhebel, ist vorzugsweise mittels eines zusätzlichen Halteelements 78a der Halteeinheit 20a (vgl. Figur 2) am Behälterunterteil 64a angeordnet, insbesondere schwenkbar am Behälterunterteil 64a gelagert. Es ist jedoch auch denkbar, dass die Verschlussklappe 14a am Behälterunterteil 64a angeordnet ist und dass das Rastelement 16a, insbesondere der schwenkbar gelagerte Spannhebel, am Behälterdeckel 62a angeordnet ist. Zudem ist alternativ oder zusätzlich denkbar, dass das Rastelement 16a, insbesondere der schwenkbar gelagerte Spannhebel, mittels der Halteeinheit 20a translatorisch beweglich gelagert ist, insbesondere mittels eines federvorgespannt beweglich gelagerten Halteelements (hier nicht dargestellt) der Halteeinheit 20a. Weitere, einem Fachmann als sinnvoll erscheinende Ausgestaltungen und/oder Anordnungen der Verschlussklappe 14a, des Rastelements 16a und/oder der Halteeinheit 20a sind ebenfalls denkbar. Die Halteelemente 22a, 24a weisen eine analoge Ausgestaltung auf, so dass eine Beschreibung eines der Halteelemente 22a, 24a für beide Halteelemente 22a, 24a gleichermaßen gilt. Die weiteren Halteelemente 40a, 42a der Halteeinheit 20a weisen eine zueinander spiegelsymmetrische Ausgestaltung auf, so dass bis auf eine teilweise spiegelsymmetrische Vertauschung eine Beschreibung eines der weiteren Halteelemente 40a, 42a für beide weiteren Halteelemente 40a, 42a gleichermaßen gilt.

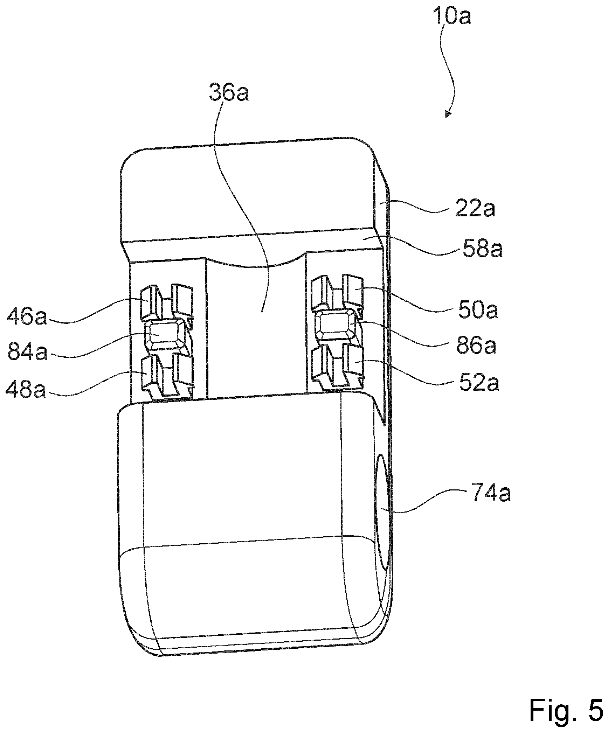

Das Halteelement 22a, 24a umfasst vorzugsweise zumindest eine Lagerausnehmung 74a, 76a (vgl. auch Figur 5), insbesondere an dem der Oberseite des Behälterdeckels 62a des Sterilbehälters 12a abgewandten Ende des Halteelements 22a, 24a, die zu einer Aufnahme des Lagerelements 72a, insbesondere des Lagerbolzens, zu einer schwenkbaren Lagerung der Verschlussklappe 14a an dem Halteelement 22a, 24a ausgebildet ist. Bevorzugt ist eine translatorische Bewegung des Halteelements 22a, 24a entlang der Bewegungsachse 26a, 28a des Halteelements 22a, 24a infolge einer Einwirkung einer Zugkraftkomponente von der Verschlussklappe 14a auf das Halteelement 22a, 24a hervorrufbar. Das Halteelement 22a, 24a ist entlang der Bewegungsachse 26a, 28a des Halteelements 22a, 24a translatorisch beweglich gelagert, die quer, insbesondere zumindest im Wesentlichen senkrecht, zur Schwenkachse 30a der Verschlussklappe 14a verläuft. Die Bewegungsachse 26a, 28a des Halteelements 22a, 24a verläuft vorzugsweise zumindest im Wesentlichen senkrecht zur Schwenkachse 66a des Rastelements 16a, insbesondere in einem am Behälterunterteil 64a angeordneten Zustand des Behälterdeckels 62a. Bevorzugt wird das Halteelement 22a, 24a zumindest während eines Überführens der Verschlussklappe 14a in oder aus der Schließstellung relativ zum Sterilbehälter 12a, insbesondere relativ zum Behälterdeckel 62a des Sterilbehälters 12a, bewegt, insbesondere translatorisch bewegt. Bevorzugt wird das Halteelement 22a, 24a zumindest während eines Inkontaktkommens oder eines Außerkontaktkommens der Verschlussklappe 14a mit dem Rastelement 16a, insbesondere des Spannhebels, translatorisch bewegt, insbesondere zusammen mit der Verschlussklappe 14a. Insbesondere ist das Halteelement 22a, 24a derart am Sterilbehälter 12a gelagert, dass das Halteelement 22a, 24a auch unabhängig von einer Bewegung der Verschlussklappe 14a, insbesondere translatorisch, beweglich relativ zum Sterilbehälter 12a ist. Die Verschlussklappe 14a ist über die translatorische Lagerung des Halteelements 22a, 24a am Sterilbehälter 12a ebenfalls, insbesondere zusätzlich zur schwenkbaren Lagerung der Verschlussklappe 14a, translatorisch relativ zum Sterilbehälter 12a gelagert. Vorzugsweise ist das Halteelement 22a, 24a an der Außenseite des Sterilbehälters 12a, insbesondere des Behälterdeckels 62a des Sterilbehälters 12a, beweglich gelagert. Bevorzugt ist das Halteelement 22a, 24a in einer zumindest im Wesentlichen parallel zu der die Seitenfläche bildenden Außenseite des Sterilbehälters 12a, insbesondere des Behälterdeckels 62a des Sterilbehälters 12a, verlaufenden Ebene translatorisch beweglich gelagert.

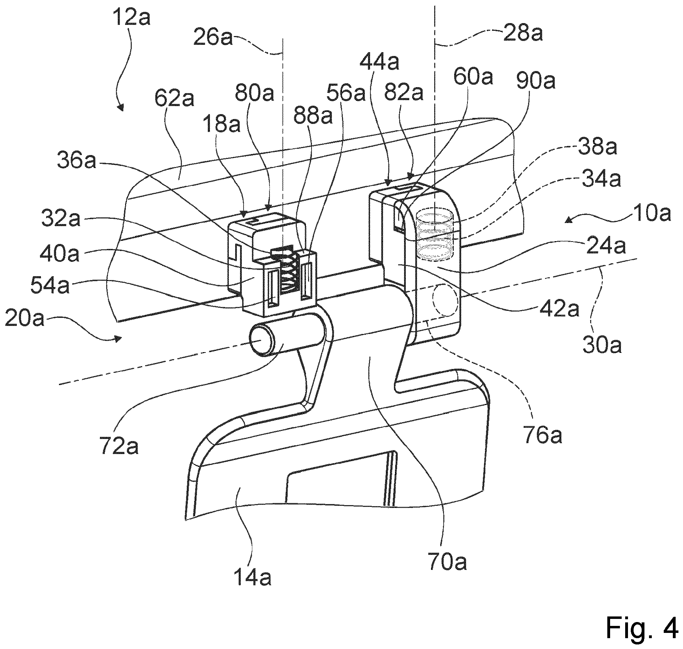

Die Halteeinheit 20a weist zumindest ein Vorspannelement 32a, 34a (vgl. Figur 4), insbesondere eine Feder, auf, das eine entlang der Bewegungsachse 26a, 28a des Halteelements 22a, 24a wirkende Vorspannkraft auf das Halteelement 22a, 24a ausübt. Das Vorspannelement 32a, 34a ist bevorzugt als Druckfeder, insbesondere als Schraubendruckfeder, ausgebildet. Es ist jedoch auch denkbar, dass das Vorspannelement 32a, 34a eine andere, einem Fachmann als sinnvoll erscheinende Ausgestaltung aufweist, wie beispielsweise eine Ausgestaltung als Zugfeder, insbesondere als Schraubenzugfeder o. dgl., als Elastomer, als Fluidzylinder, insbesondere als Öldruckfeder, als Gasfeder od. dgl., als Tellerfeder o. dgl. Das Vorspannelement 32a, 34a stützt sich mit einem Ende an dem Halteelement 22a, 24a ab und mit einem weiteren Ende stützt sich das Vorspannelement 32a, 34a an dem weiteren Halteelement 40a, 42a oder an dem Behälterdeckel 62a des Sterilbehälters 12a ab. Das Vorspannelement 32a, 34a bewirkt in der Schließstellung der Verschlussklappe 14a ein Ausüben einer Zugkraft der Verschlussklappe 14a auf das Rastelement 16a, insbesondere eine in Richtung des Behälterdeckels 62a des Sterilbehälters 12a wirkende Zugkraft. Bevorzugt weist die Halteeinheit 20a zumindest zwei Vorspannelemente 32a, 34a auf, die eine analoge Ausgestaltung aufweisen, so dass eine Beschreibung eines der Vorspannelemente 32a, 34a für beide Vorspannelemente 32a, 34a gleichermaßen gilt.

Das Halteelement 22a, 24a begrenzt einen Hohlraum 36a, 38a zu einer Aufnahme des Vorspannelements 32a, 34a der Halteeinheit 20a zumindest teilweise, wobei das Vorspannelement 32a, 34a zumindest teilweise von dem Halteelement 22a, 24a umgeben wird und sich an dem Halteelement 22a, 24a abstützt (vgl. Figuren 4 und 5). Die Halteeinheit 20a weist zumindest das Vorspannelement 32a, 34a zu einem Ausüben einer entlang der Bewegungsachse 26a, 28a des Halteelements 22a, 24a wirkenden Vorspannkraft auf das Halteelement 22a, 24a und zumindest das die Befestigungsschnittstelle 18a, 44a umfassende weitere Halteelement 40a, 42a auf, wobei das Halteelement 22a, 24a und das weitere Halteelement 40a, 42a zusammen das Vorspannelement 32a, 34a zumindest im Wesentlichen vollständig umschließen (vgl. Figur 4). Die Befestigungsschnittstelle 18a, 44a ist vorzugsweise als Formschlussschnittstelle ausgebildet, die mit einer am Behälterdeckel 62a angeordneten, insbesondere einteilig mit dem Behälterdeckel 62a ausgebildeten, Gegenformschlussschnittstelle 80a, 82a des Sterilbehälters 12a zu einer Fixierung des weiteren Halteelements 40a, 42a zusammenwirkt. Vorzugsweise wird das weitere Halteelement 40a, 42a infolge eines Einwirkens der Vorspannkraft des Vorspannelements 32a, 34a auf das Halteelement 22a, 24a und das weitere Halteelement 40a, 42a in einer Endposition der Befestigungsschnittstelle 18a, 44a gehalten. Es ist jedoch auch denkbar, dass in einer alternativen Ausgestaltung die Halteeinheit 20a unabhängig von dem weiteren Halteelement 40a, 42a ausgebildet ist und die Befestigungsschnittstelle 18a, 44a am Halteelement 22a, 24a angeordnet ist, die zugleich eine Lagerschnittstelle zu einer translatorischen Lagerung des Halteelements 22a, 24a bildet. Weitere, einem Fachmann als sinnvoll erscheinende Ausgestaltungen oder Positionierungen der Befestigungsschnittstelle 18a, 44a zu einer verliersicheren Anordnung des Halteelements 22a, 24a und/oder des weiteren Halteelements 40a, 42a an dem Sterilbehälter 12a sind ebenfalls denkbar, wie beispielsweise eine Ausgestaltung der Befestigungsschnittstelle 18a, 44a als Niet- oder Schraubenschnittstelle zu einer Befestigung zumindest des weiteren Halteelements 40a, 42a an dem Sterilbehälter 12a, insbesondere an dem Behälterdeckel 62a.

Die Halteeinheit 20a weist zumindest ein Sicherungselement 46a, 48a, 50a, 52a (vgl. Figur 5) auf, das das Halteelement 22a, 24a weitestgehend gegen eine Bewegung entlang einer quer, insbesondere zumindest im Wesentlichen senkrecht, zur Bewegungsachse 26a, 28a des Halteelements 22a, 24a verlaufenden Richtung sichert. Das Sicherungselement 46a, 48a, 50a, 52a ist vorzugsweise zu einer form- und/oder kraftschlüssigen Verbindung mit einem weiteren Element, insbesondere einer Führungsnut 54a, 56a, der Halteeinheit 20a vorgesehen. Insbesondere ist das Sicherungselement 46a, 48a, 50a, 52a als federelastischer Rastclip, als federelastischer Rasthaken, als federelastischer Rastfortsatz oder als ein anderes, einem Fachmann als sinnvoll erscheinendes Sicherungselement ausgebildet. Bevorzugt umfasst die Halteeinheit 20a eine Vielzahl an Sicherungselementen 46a, 48a, 50a, 52a, die das Halteelement 22a, 24a weitestgehend gegen eine Bewegung entlang der quer, insbesondere zumindest im Wesentlichen senkrecht, zur Bewegungsachse 26a, 28a des Halteelements 22a, 24a verlaufenden Richtung sichern. Insbesondere umfasst die Halteeinheit 20a zumindest zwei und bevorzugt zumindest vier Sicherungselemente 46a, 48a, 50a, 52a, die das Halteelement 22a, 24a weitestgehend gegen eine Bewegung entlang der quer, insbesondere zumindest im Wesentlichen senkrecht, zur Bewegungsachse 26a, 28a des Halteelements 22a, 24a verlaufenden Richtung sichern, insbesondere infolge eines Zusammenwirkens mit zumindest einem weiteren Element der Halteeinheit 20a, insbesondere mit zumindest einer Führungsnut 54a, 56a der Halteeinheit 20a. Vorzugsweise ist das Sicherungselement 46a, 48a, 50a, 52a bzw. sind die Sicherungselemente 46a, 48a, 50a, 52a versetzt zur Bewegungsachse 26a, 28a des Halteelements 22a, 24a am Halteelement 22a, 24a angeordnet, insbesondere einteilig mit dem Halteelement 22a, 24a ausgebildet (vgl. Figur 5).

Die Halteeinheit 20a umfasst vorzugsweise zumindest ein Führungselement 84a, 86a, das zu einer Führung des Halteelements 22a, 24a während einer Bewegung des Halteelements 22a, 24a relativ zum Sterilbehälter 12a und/oder zur Verschlussklappe 14a vorgesehen ist (vgl. Figur 5). Das Führungselement 84a, 86a ist insbesondere einteilig mit dem Halteelement 22a, 24a ausgebildet. Das Führungselement 84a, 86a ist als Führungsfortsatz oder als Führungszapfen ausgebildet, der sich zumindest im Wesentlichen senkrecht zur Bewegungsachse 26a, 28a des Halteelements 22a, 24a erstreckt. Vorzugsweise ist das Führungselement 84a, 86a, betrachtet entlang einer zumindest im Wesentlichen parallel zur Bewegungsachse 26a, 28a verlaufenden Richtung zwischen zwei der Sicherungselemente 46a, 48a, 50a, 52a am Halteelement 22a, 24a angeordnet. Bevorzugt weist die Halteeinheit 20a zumindest zwei Führungselemente 84a, 86a pro Halteelement 22a, 24a auf. Die Führungselemente 84a, 86a sind vorzugsweise seitlich versetzt zur Bewegungsachse 26a, 28a und/oder zu dem von dem Halteelement 22a, 24a zumindest teilweise begrenzten Hohlraum 36a, 38a am Halteelement 22a, 24a angeordnet (vgl. Figur 5).

Die Halteeinheit 20a weist zumindest das Sicherungselement 46a, 48a, 50a, 52a auf, das an dem Halteelement 22a, 24a angeordnet ist, insbesondere einteilig mit dem Halteelement 22a, 24a ausgebildet ist, und in die, insbesondere an dem weiteren Halteelement 40a, 42a der Halteeinheit 20a, angeordnete Führungsnut 54a, 56a der Halteeinheit 20a eingreift. Vorzugsweise weist jedes der weiteren Halteelemente 40a, 42a zumindest zwei Führungsnuten 54a, 56a auf (in Figur 4 sind lediglich zwei Führungsnuten 54a, 56a eines der weiteren Halteelemente 40a, 42a zu sehen). Die Führungsnuten 54a, 56a sind vorzugsweise seitlich versetzt zur Bewegungsachse 26a, 28a und/oder zu dem von dem weiteren Halteelement 40a, 42a zumindest teilweise begrenzten Hohlraum 36a, 38a am weiteren Halteelement 40a, 42a angeordnet (vgl. Figur 4). Die Führungsnuten 54a, 56a weisen eine analoge Ausgestaltung auf, so dass eine Beschreibung einer der Führungsnuten 54a, 56a gleichermaßen für die andere der beiden Führungsnuten 54a, 56a zu verstehen ist. Vorzugsweise rastet das Sicherungselement 46a, 48a, 50a, 52a in die Führungsnut 54a, 56a ein. Eine Längserstreckungsachse der Führungsnut 54a, 56a verläuft vorzugsweise zumindest im Wesentlichen parallel zur Bewegungsachse 26a, 28a des Halteelements 22a, 24a. Die Führungsnut 54a, 56a weist zwei geschlossene Enden auf. Die Führungsnut 54a, 56a weist eine Ausgestaltung gemäß einem Langloch auf. Vorzugsweise ist durch ein Zusammenwirken der mit geschlossenen Enden ausgebildeten Führungsnut 54a, 56a mit den Sicherungselementen 46a, 48a, 50a, 52a und/oder den Führungselementen 84a, 86a eine maximale Bewegungsstrecke des Halteelements 22a, 24a relativ zum weiteren Halteelement 40a, 42a und/oder zum Sterilbehälter 12a begrenzbar.

Die Halteeinheit 20a weist zumindest das an dem Halteelement 22a, 24a angeordnete Sicherungselement 46a, 48a, 50a, 52a und zumindest die, insbesondere an dem weiteren Halteelement 40a, 42a der Halteeinheit 20 angeordnete, Führungsnut 54a, 56a auf, die infolge eines Zusammenwirkens mit dem Sicherungselement 46a, 48a, 50a, 52a und/oder mit dem Führungselement 84a, 86a zu einer Führung einer Bewegung des Halteelements 22a, 24a entlang der Bewegungsachse 26a, 28a des Halteelements 22a, 24a vorgesehen ist. Insbesondere wirkt das Sicherungselement 46a, 48a, 50a, 52a derart form- und/oder kraftschlüssig mit der Führungsnut 54a, 56a zusammen, dass ein Bewegung des Sicherungselements 46a, 48a, 50a, 52a entlang der Längserstreckungsachse der Führungsnut 54a, 56a durchführbar ist und eine/r Bewegung des Sicherungselements 46a, 48a, 50a, 52a entlang einer quer, insbesondere zumindest im Wesentlichen senkrecht, zur Längserstreckungsachse infolge eines Formschlusses, insbesondere infolge eines Hintergreifens des Sicherungselements 46a, 48a, 50a, 52a von zumindest einem Rand der Führungsnut 54a, 56a, entgegengewirkt oder weitestgehend vermieden wird. Vorzugsweise bilden die Sicherungselemente 46a, 48a, 50a, 52a zusammen mit der Führungsnut 54a, 56a eine Lagerungsschnittstelle der Halteeinheit 20a zu einer beweglichen Lagerung des Halteelements 22a, 24a relativ zum Sterilbehälter 12a.

Das Halteelement 22a, 24a weist zumindest einen Anschlagsfortsatz 58a, 60a zu einer Begrenzung einer maximalen Bewegungsstrecke des Halteelements 22a, 24a auf, der zu einem Zusammenwirken mit dem weiteren Halteelement 40a, 42a der Halteeinheit 20a oder in einer alternativen, hier nicht dargestellten Ausführung mit einem Fortsatz, insbesondere des Behälterdeckels 62a, des Sterilbehälters 12a vorgesehen ist. Der Anschlagsfortsatz 58a, 60a ist zu einer Begrenzung einer maximalen Bewegungsstrecke des Halteelements 22a, 24a relativ zum weiteren Halteelement 40a, 42a entlang einer zumindest im Wesentlichen parallel zur Bewegungsachse 26a, 28a verlaufenden Richtung in Richtung des Behälterunterteils 64a vorgesehen. Vorzugsweise bildet der Anschlagsfortsatz 58a, 60a eine Anschlagfläche, die mit einer Gegenanschlagfläche 88a, 90a des weiteren Halteelements 40a, 42a oder des Sterilbehälters 12a, insbesondere des Behälterdeckels 62a, zu einer Begrenzung der maximalen Bewegungsstrecke des Halteelements 22a, 24a entlang der Bewegungsachse 26a, 28a des Halteelements 22a, 24a zusammenwirkt. Bevorzugt ist die Anschlagfläche des Anschlagsfortsatzes 58a, 60a einteilig mit einer Abstützfläche des Halteelements 22a, 24a, an der sich das Vorspannelement 32a, 34a mit einem Ende an dem Halteelement 22a, 24a abstützt, ausgebildet.

Mittels der Verschlussvorrichtung 10a kann vorteilhaft ein hoher Betätigungskomfort und/oder eine hohe Verschlussspannkraft realisiert werden, da insbesondere das Rastelement 16a durch die Verschlussklappe 14a, welche durch die Vorspannkraft des Vorspannelements 32a, 34a in Richtung des Behälterdeckels 62a mit einer Zugkraft beaufschlagt ist, über den Totpunkt des Rastelements 16a hinweg in Richtung des Sterilbehälters 12a mit einer Kraft beaufschlagt ist, so dass das Rastelement 16a sicher in der Schließstellung gehalten werden kann. Es kann vorteilhaft eine zusätzliche Bewegung der Verschlussklappe 14a realisiert werden, um eine komfortable Überführung der Verschlussklappe 14a in die Schließstellung oder aus der Schließstellung zu ermöglichen, insbesondere infolge einer zusätzlich zur Schwenkbewegung realisierbaren Linearbewegung durch die translatorisch bewegliche Lagerung des Halteelements 22a, 24a, an dem die Verschlussklappe 14a schwenkbar gelagert ist. Es kann konstruktiv einfach eine Bewegungsüberlagerung einer Rotations- und einer Translationsbewegung realisiert werden, um ein komfortables Eingreifen oder aus einem Eingriff Bringen des Rastelements 16a und der Verschlussklappe 14a zu ermöglichen.

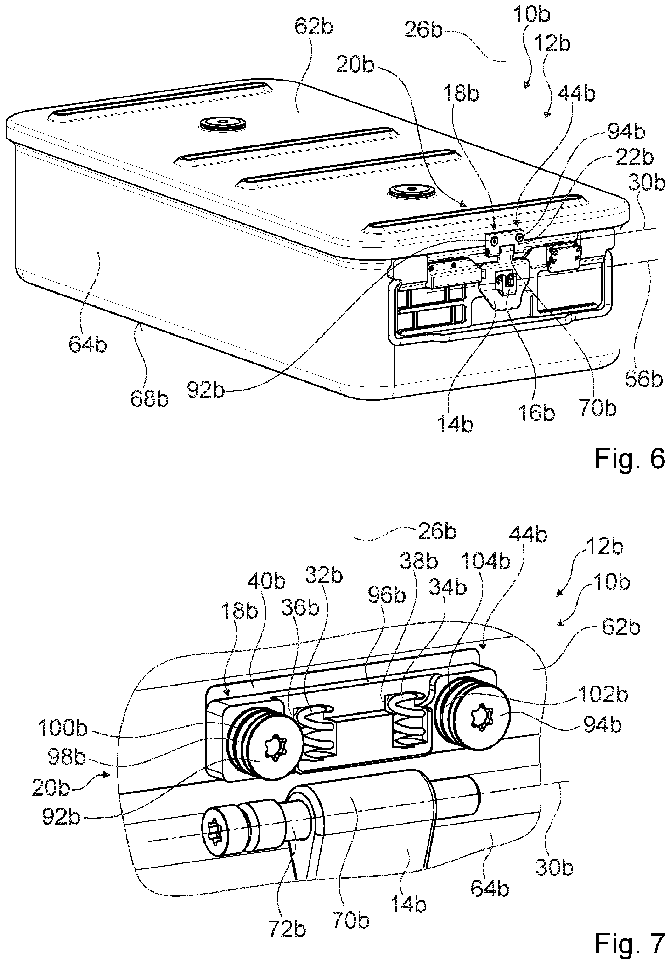

In den Figuren 6 bis 8b ist ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 5, verwiesen werden kann. Zur Unterscheidung der Ausführungsbeispiele ist der Buchstabe a den Bezugszeichen des Ausführungsbeispiels in den Figuren 1 bis 5 nachgestellt. In den Ausführungsbeispielen der Figuren 6 bis 8b ist der Buchstabe a durch den Buchstaben b ersetzt.

Figur 6 zeigt einen alternativen Sterilbehälter 12b mit zumindest einer alternativen Verschlussvorrichtung 10b. Der Sterilbehälter 12b ist als medizinischer Sterilbehälter ausgebildet. Der Sterilbehälter 12b umfasst einen Behälterdeckel 62b und ein Behälterunterteil 64b. Das Behälterunterteil 64b ist vorzugsweise kastenartig ausgebildet und begrenzt einen Aufnahmeraum. Das Behälterunterteil 64b ist mittels des Behälterdeckels 62b auf eine, einem Fachmann bereits bekannte Art und Weise, insbesondere hermetisch, verschließbar. Das Behälterunterteil 64b ist zur Bildung eines geschlossenen Sterilraums mittels des Behälterdeckels 62b verschließbar. Der Behälterdeckel 62b ist abnehmbar an dem Behälterunterteil 64b angeordnet und mittels umlaufend zwischen dem Behälterdeckel 62b und dem Behälterunterteil 64b angeordneten Dichtungsmitteln luftdicht auf das Behälterunterteil 64b aufsetzbar und mit diesem verbindbar.

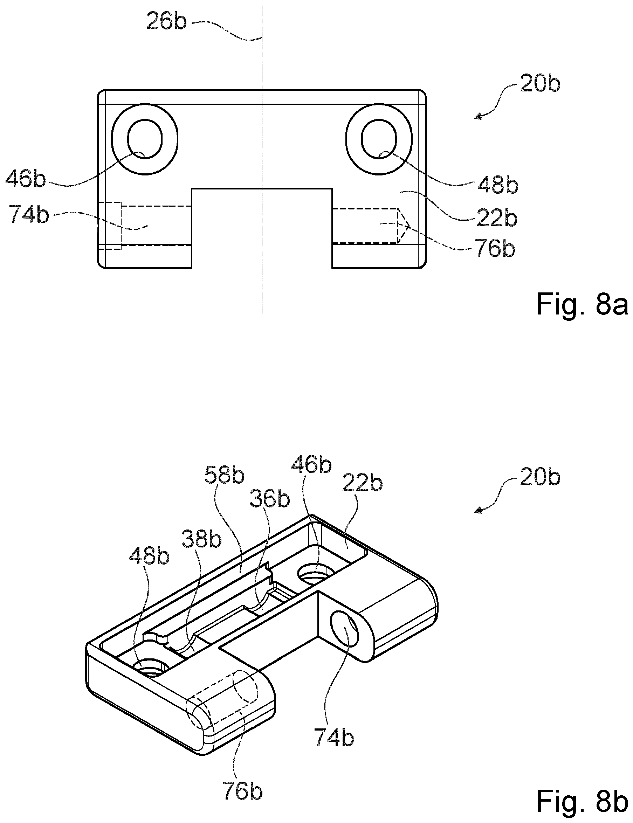

Die Verschlussvorrichtung 10b für den Sterilbehälter 12b umfasst zumindest eine schwenkbar gelagerte Verschlussklappe 14b, zumindest ein zumindest in einer Schließstellung mit der Verschlussklappe 14b, insbesondere form- und/oder kraftschlüssig, zusammenwirkendes Rastelement 16b, insbesondere einen schwenkbar gelagerten Spannhebel, und zumindest eine zumindest eine Befestigungsschnittstelle 18b, 44b zu einem Befestigen an dem Sterilbehälter 12b aufweisende Halteeinheit 20b zu einem Halten der, insbesondere schwenkbar an der Halteeinheit 20b gelagerten, Verschlussklappe 14b und/oder des Rastelements 16b an dem Sterilbehälter 12b. Das Halteelement 22b ist entlang einer Bewegungsachse 26b des Halteelements 22b translatorisch beweglich gelagert, wobei die Bewegungsachse 26b des Halteelements 22b quer, insbesondere zumindest im Wesentlichen senkrecht, zu einer Schwenkachse 30b der Verschlussklappe 14b verläuft. Das Halteelement 22b weist vorzugsweise zwei Lagerfortsätze auf, in denen jeweils eine Lagerausnehmung 74b, 76b angeordnet ist (vgl. Figuren 8a und 8b). Die Lagerausnehmungen 74b, 76b sind zu einer Aufnahme eines Lagerelements 72b (vgl. Figur 7) zu einer schwenkbaren Lagerung der Verschlussklappe 14b an dem Halteelement 22b vorgesehen. Die Lagerausnehmungen 74b, 76b können identisch ausgebildet sein, insbesondere als Durchgangsöffnungen, oder verschieden voneinander ausgebildet sein, insbesondere eine der Lagerausnehmungen 74b, 76b kann als Klemmausnehmung oder als Schraubausnehmung zu einer Sicherung des Lagerelements 72b ausgebildet sein und eine andere der Lagerausnehmungen 74b, 76b kann als Durchgangsöffnung ausgebildet sein. Die Lagerausnehmungen 74b, 76b weisen vorzugsweise quer, insbesondere zumindest im Wesentlichen senkrecht zur Bewegungsachse 26b des Halteelements 22b verlaufende Längsachsen auf.

Die Halteeinheit 20b weist zumindest ein Vorspannelement 32b, 34b, insbesondere eine Feder, auf, das eine entlang einer zumindest im Wesentlichen parallel zu der Bewegungsachse 26b des Halteelements 22b wirkende Vorspannkraft auf das Halteelement 22b ausübt (vgl. Figur 7). Vorzugsweise weist die Halteeinheit 20b zumindest zwei Vorspannelemente 32b, 34b, insbesondere zwei Schraubenfedern, auf, die jeweils eine entlang einer zumindest im Wesentlichen parallel zu der Bewegungsachse 26b des Halteelements 22b wirkende Vorspannkraft auf das Halteelement 22b ausüben. Das Halteelement 22b begrenzt vorzugsweise zumindest teilweise einen Hohlraum 36b, 38b, insbesondere zumindest zwei Hohlräume 36b, 38b (vgl. Figuren 7 sowie 8a und 8b), zu einer Aufnahme des Vorspannelements 32b, 34b, insbesondere der Vorspannelemente 32b, 34b, wobei das Vorspannelement 32b, 34b, insbesondere die Vorspannelemente 32b, 34b, zumindest teilweise von dem Halteelement 22b umgeben wird/werden und sich an dem Halteelement 22b abstützt/abstützen. Die Halteeinheit 20b umfasst zumindest ein die Befestigungsschnittstelle 18b, 44b zumindest teilweise umfassendes und/oder bildendes weiteres Halteelement 40b aufweist, wobei das Halteelement 22b und das weitere Halteelement 40b zusammen das Vorspannelement 32b, 34b, insbesondere die Vorspannelemente 32b, 34b, zumindest im Wesentlichen vollständig umschließen. Das weitere Halteelement 40b ist vorzugsweise einteilig mit dem Sterilbehälter 12b, insbesondere mit dem Behälterdeckel 62b des Sterilbehälters 12b, ausgebildet.

Die Halteeinheit 20b weist zumindest ein Sicherungselement 46b, 48b auf, das das Halteelement 22b weitestgehend gegen eine Bewegung entlang einer quer, insbesondere zumindest im Wesentlichen senkrecht, zur Bewegungsachse 26b des Halteelements 22b verlaufenden Richtung sichert (vgl. Figuren 8a und 8b). Das Sicherungselement 46b, 48b ist vorzugsweise als Langloch ausgebildet. Das Sicherungselement 46b, 48b ist vorzugsweise an dem Halteelement 22b angeordnet, insbesondere in das Halteelement 22b eingebracht. Die Halteeinheit 20b weist zumindest zwei Sicherungselemente 46b, 48b auf, die das Halteelement 22b weitestgehend gegen eine Bewegung entlang der quer, insbesondere zumindest im Wesentlichen senkrecht, zur Bewegungsachse 26b des Halteelements 22b verlaufenden Richtung sichern. Die Sicherungselemente 46b, 48b weisen vorzugsweise eine analoge Ausgestaltung auf. Das/die Sicherungselement/e 46b, 48b bildet/bilden vorzugsweise einen Teil der Befestigungsschnittstelle 18b, 44b. Die Befestigungsschnittstelle 18b, 44b weist das zumindest eine als Langloch ausgebildete Sicherungselement 46b, 48b auf, das an dem Halteelement 22b angeordnet ist, insbesondere einteilig mit dem Halteelement 22b ausgebildet ist, und mit zumindest einem Befestigungselement 92b, 94b der Befestigungsschnittstelle 18b, 44b derart zusammenwirkt, dass eine Führung einer Bewegung des Halteelements 22b entlang der Bewegungsachse 26b des Halteelements 22b erfolgt. Die Befestigungsschnittstelle 18b, 44b wird vorzugsweise von den zwei einteilig mit dem Halteelement 22b und als Langlöchern ausgebildeten Sicherungselementen 46b, 48b, dem weiteren Halteelement 40b und den zumindest zwei Befestigungselementen 92b, 94b gebildet. Das/die Befestigungselement/e 92b, 94b ist/sind bevorzugt als Befestigungsbolzen, insbesondere als Schraube/n, ausgebildet. Vorzugsweise wirkt jeweils ein Randbereich der als Langlöcher ausgebildeten Sicherungselemente 46b, 48b derart mit einem Schraubenkopf der jeweils als Schraube ausgebildeten Befestigungselemente 92b, 94b zusammen, dass eine Sicherung einer Bewegung des Halteelements 22b entlang der quer, insbesondere zumindest im Wesentlichen senkrecht, zur Bewegungsachse 26b des Halteelements 22b verlaufenden Richtung erfolgt. Vorzugsweise wirkt jeweils der Randbereich des Langlochs derart mit einem Schraubenschaft der jeweils als Schraube ausgebildeten Befestigungselemente 92b, 94b zusammen, dass eine Führung einer Bewegung des Halteelements 22b entlang der Bewegungsachse 26b des Halteelements 22b erfolgt. Das Halteelement 22b weist zumindest einen Anschlagsfortsatz 58b (vgl. Figur 8b) zu einer Begrenzung einer maximalen Bewegungsstrecke des Halteelements 22b auf, der zu einem Zusammenwirken mit einem Fortsatz 96b, insbesondere des Behälterdeckels 62b, des Sterilbehälters 12b vorgesehen ist. Das weitere Halteelement 40b ist vorzugsweise einteilig mit dem Sterilbehälter 12b, insbesondere mit dem Behälterdeckel 62b des Sterilbehälters 12b, ausgebildet. Bevorzugt wird der Fortsatz 96b von dem weiteren Halteelement 40b gebildet (vgl. Figur 7).

Die Halteeinheit 20b weist zumindest ein Gleitelement 98b, 100b, 102b, 104b auf, das zu einer Gleitlagerung des Halteelements 22b an dem, insbesondere einteilig mit dem Sterilbehälter 12b ausgebildeten, weiteren Halteelement 40b der Halteeinheit 20b vorgesehen ist (vgl. Figur 7). Vorzugsweise weist das Gleitelement 98b, 100b, 102b, 104b eine Oberfläche mit reibungsmindernden Eigenschaften auf. Beispielsweise ist denkbar, dass das Gleitelement 98b, 100b, 102b, 104b selbst aus einem Werkstoff mit geringem Reibwert hergestellt ist, wie beispielsweise Graphit, Polyamid, Polyoxymethylen o. dgl., dass das Gleitelement 98b, 100b, 102b, 104b eine Oberflächenbeschichtung aus einem Werkstoff mit geringem Reibwert aufweist oder dass das Gleitelement 98b, 100b, 102b, 104b Gleitfluideinlagerungen oder Aufnahmen für Gleitfluide aufweist. Bevorzugt umfasst die Halteeinheit 20b eine Vielzahl an Gleitelementen 98b, 100b, 102b, 104b, die zu einer Gleitlagerung des Halteelements 22b an dem, insbesondere einteilig mit dem Sterilbehälter 12b ausgebildeten, weiteren Halteelement 40b der Halteeinheit 20b vorgesehen sind. Bevorzugt sind zumindest zwei der Gleitelemente 98b, 100b, 102b, 104b, insbesondere räumlich, zwischen dem Halteelement 22b und dem weiteren Halteelement 40b angeordnet, insbesondere entlang einer quer zur Bewegungsachse 26b des Halteelements 22b verlaufenden Richtung betrachtet. Vorzugsweise sind zumindest zwei weitere der Gleitelemente 98b, 100b, 102b, 104b, insbesondere räumlich, zwischen dem Halteelement 22b und den zwei Befestigungselementen 92b, 94b angeordnet, insbesondere entlang der quer zur Bewegungsachse 26b des Halteelements 22b verlaufenden Richtung betrachtet. Die Gleitelemente 98b, 100b, 102b, 104b, insbesondere jeweils zwei der Gleitelemente 98b, 100b, 102b, 104b, umgeben vorzugsweise die Befestigungselemente 92b, 94b zu einem Großteil, insbesondere vollständig, entlang einer Umfangsrichtung, die in einer sich senkrecht zu einer Längsachse der Befestigungselemente 92b, 94b erstreckenden Ebene verläuft. Das/die Gleitelement/e 98b, 100b, 102b, 104b weist/weisen vorzugsweise einen kreisringförmigen Querschnitt auf. Es ist jedoch auch denkbar, dass das/die Gleitelement/e 98b, 100b, 102b, 104b einen anderen, einem Fachmann als sinnvoll erscheinenden Querschnitt aufweist/aufweisen, wie beispielsweise sichelförmig, polygonal o. dgl. Bevorzugt ist/sind das Gleitelement/e 98b, 100b, 102b, 104b abgeschirmt an dem Halteelement 22b und/oder an dem weiteren Halteelement 40b angeordnet, wie beispielsweise mittels einer Anordnung zumindest eines Dichtlippenelements (hier nicht näher dargestellt) zwischen dem Halteelement 22b und dem weiteren Halteelement 40b zu einem Entgegenwirken eines Eindringens von Schmutz.

### Bezugszeichen

- 10: Verschlussvorrichtung
- 12: Sterilbehälter
- 14: Verschlussklappe
- 16: Rastelement
- 18: Befestigungsschnittstelle
- 20: Halteeinheit
- 22: Halteelement
- 24: Halteelement
- 26: Bewegungsachse
- 28: Bewegungsachse
- 30: Schwenkachse
- 32: Vorspannelement
- 34: Vorspannelement
- 36: Hohlraum
- 38: Hohlraum
- 40: Halteelement
- 42: Halteelement
- 44: Befestigungsschnittstelle
- 46: Sicherungselement
- 48: Sicherungselement
- 50: Sicherungselement
- 52: Sicherungselement
- 54: Führungsnut
- 56: Führungsnut
- 58: Anschlagsfortsatz
- 60: Anschlagsfortsatz
- 62: Behälterdeckel
- 64: Behälterunterteil
- 66: Schwenkachse
- 68: Bodenstandfläche
- 70: Lagerfortsatz
- 72: Lagerelement
- 74: Lagerausnehmung
- 76: Lagerausnehmung
- 78: Halteelement
- 80: Gegenformschlussschnittstelle
- 82: Gegenformschlussschnittstelle
- 84: Führungselement
- 86: Führungselement
- 88: Gegenanschlagfläche
- 90: Gegenanschlagfläche
- 92: Befestigungselement
- 94: Befestigungselement
- 96: Fortsatz
- 98: Gleitelement
- 100: Gleitelement
- 102: Gleitelement
- 104: Gleitelement

## Patentansprüche

1. Verschlussvorrichtung für Sterilbehälter, mit zumindest einer schwenkbar gelagerten Verschlussklappe (14a; 14b), mit zumindest einem zumindest in einer Schließstellung mit der Verschlussklappe (14a; 14b), insbesondere form- und/oder kraftschlüssig, zusammenwirkenden Rastelement (16a; 16b), insbesondere einem schwenkbar gelagerten Spannhebel, und mit zumindest einer zumindest eine Befestigungsschnittstelle (18a, 44a; 18b, 44b) zu einem Befestigen an dem Sterilbehälter aufweisenden Halteeinheit (20a; 20b) zu einem Halten der, insbesondere schwenkbar an der Halteeinheit (20a; 20b) gelagerten, Verschlussklappe (14a; 14b) und/oder des Rastelements (16a; 16b) an dem Sterilbehälter, **dadurch gekennzeichnet, dass** die Halteeinheit (20a; 20b) zumindest ein, insbesondere translatorisch, beweglich gelagertes Halteelement (22a, 24a; 22b) aufweist, das separat zur Verschlussklappe (14a; 14b) ausgebildet ist und derart beweglich gelagert ist, dass das Halteelement (22a, 24a; 22b) zumindest während eines Überführens der Verschlussklappe (14a; 14b) in oder aus der Schließstellung bewegbar ist.

2. Verschlussvorrichtung nach Anspruch 1, **dadurch gekennzeichnet, dass** das Halteelement (22a, 24a; 22b) entlang einer Bewegungsachse (26a, 28a; 26b) des Halteelements (22a, 24a; 22b) translatorisch beweglich gelagert ist, die quer, insbesondere zumindest im Wesentlichen senkrecht, zu einer Schwenkachse (30a; 30b) der Verschlussklappe (14a; 14b) verläuft.

3. Verschlussvorrichtung nach Anspruch 1 oder 2, **dadurch gekennzeichnet, dass** die Halteeinheit (20a; 20b) zumindest ein Vorspannelement (32a, 34a; 32b, 34b), insbesondere eine Feder, aufweist, das eine entlang einer Bewegungsachse (26a, 28a; 26b) des Halteelements (22a, 24a; 22b) wirkende Vorspannkraft auf das Halteelement (22a, 24a; 22b) ausübt.

4. Verschlussvorrichtung nach einem der vorhergehenden Ansprüche, **dadurch gekennzeichnet, dass** das Halteelement (22a, 24a; 22b) einen Hohlraum (36a, 38a; 36b, 38b) zu einer Aufnahme eines Vorspannelements (32a, 34a; 32b, 34b) der Halteeinheit (20a; 20b) zumindest teilweise begrenzt, wobei das Vorspannelement (32a, 34a; 32b, 34b) zumindest teilweise von dem Halteelement (22a, 24a; 22b) umgeben wird und sich an dem Halteelement (22a, 24a; 22b) abstützt.

5. Verschlussvorrichtung nach einem der vorhergehenden Ansprüche, **dadurch gekennzeichnet, dass** die Halteeinheit (20a; 20b) zumindest ein Vorspannelement (32a, 34a; 32b, 34b) zu einem Ausüben einer entlang einer Bewegungsachse (26a, 28a; 26b) des Halteelements (22a, 24a; 22b) wirkenden Vorspannkraft auf das Halteelement (22a, 24a; 22b) und zumindest ein die Befestigungsschnittstelle (18a, 44a; 18b, 44b) umfassendes weiteres Halteelement (40a, 42a; 40b) aufweist, wobei das Halteelement (22a, 24a; 22b) und das weitere Halteelement (40a, 42a; 40b) zusammen das Vorspannelement (32a, 34a; 32b, 34b) zumindest im Wesentlichen vollständig umschließen.

6. Verschlussvorrichtung nach einem der vorhergehenden Ansprüche, **dadurch gekennzeichnet, dass** die Halteeinheit (20a; 20b) zumindest ein Sicherungselement (46a, 48a, 50a, 52a; 46b, 48b) aufweist, das das Halteelement (22a, 24a; 22b) weitestgehend gegen eine Bewegung entlang einer quer, insbesondere zumindest im Wesentlichen senkrecht, zu einer Bewegungsachse (26a, 28a; 26b) des Halteelements (22a, 24a; 22b) verlaufenden Richtung sichert.

7. Verschlussvorrichtung nach einem der vorhergehenden Ansprüche, **dadurch gekennzeichnet, dass** die Halteeinheit (20a) zumindest ein Sicherungselement (46a, 48a, 50a, 52a) aufweist, das an dem Halteelement (22a, 24a) angeordnet ist, insbesondere einteilig mit dem Halteelement (22a, 24a) ausgebildet ist, und in eine, insbesondere an einem weiteren Halteelement (40a, 42a) der Halteeinheit (20a), angeordnete Führungsnut (54a, 56a) der Halteeinheit (20a) eingreift.

8. Verschlussvorrichtung nach einem der vorhergehenden Ansprüche, **dadurch gekennzeichnet, dass** die Halteeinheit (20a) zumindest ein an dem Halteelement (22a, 24a) angeordnetes Sicherungselement (46a, 48a, 50a, 52a) und zumindest eine, insbesondere an einem weiteren Halteelement (40a, 42a) der Halteeinheit (20a) angeordnete, Führungsnut (54a, 56a) aufweist, die infolge eines Zusammenwirkens mit dem Sicherungselement (46a, 48a, 50a, 52a) zu einer Führung einer Bewegung des Halteelements (22a, 24a) entlang einer Bewegungsachse (26a, 28a) des Halteelements (22a, 24a) vorgesehen ist.

9. Verschlussvorrichtung nach einem der vorhergehenden Ansprüche, **dadurch gekennzeichnet, dass** die Befestigungsschnittstelle (18b, 44b) zumindest ein als Langloch ausgebildetes Sicherungselement (46b, 48b) aufweist, das an dem Halteelement (22b) angeordnet ist, insbesondere einteilig mit dem Halteelement (22b) ausgebildet ist, und mit zumindest einem Befestigungselement (92b, 94b) der Befestigungsschnittstelle (18b, 44b) derart zusammenwirkt, dass eine Führung einer Bewegung des Halteelements (22b) entlang einer Bewegungsachse (26b) des Halteelements (22b) erfolgt.

10. Verschlussvorrichtung nach einem der vorhergehenden Ansprüche, **dadurch gekennzeichnet, dass** die Halteeinheit (20b) zumindest ein Gleitelement (98b, 100b, 102b, 104b) aufweist, das zu einer Gleitlagerung des Halteelements (22b) an einem, insbesondere einteilig mit dem Sterilbehälter ausgebildeten, weiteren Halteelement (40b) der Halteeinheit (20b) vorgesehen ist.

11. Verschlussvorrichtung nach einem der vorhergehenden Ansprüche, **dadurch gekennzeichnet, dass** das Halteelement (22a, 24a; 22b) zumindest einen Anschlagsfortsatz (58a, 60a; 58b) zu einer Begrenzung einer maximalen Bewegungsstrecke des Halteelements (22a, 24a; 22b) aufweist, der zu einem Zusammenwirken mit einem weiteren Halteelement (40a, 42a) der Halteeinheit (20a) oder mit einem Fortsatz (96b), insbesondere eines Behälterdeckels (62b), des Sterilbehälters vorgesehen ist.

12. Sterilbehälter mit zumindest einer Verschlussvorrichtung nach einem der vorhergehenden Ansprüche.
